(19) Europäisches Patentamt
European Patent Office
Office européen des brevets

(11) EP 3 293 240 B1

(12) # EUROPEAN PATENT SPECIFICATION

(45) Date of publication and mention of the grant of the patent:
**08.04.2020 Bulletin 2020/15**

(21) Application number: **16789562.2**

(22) Date of filing: **02.05.2016**

(51) Int Cl.:
***C09K 3/00*** *(2006.01)*
***A23L 3/37*** *(2006.01)*
***C07K 5/065*** *(2006.01)*
***C07K 5/087*** *(2006.01)*
***C07K 5/107*** *(2006.01)*
***C07K 7/06*** *(2006.01)*
***C09D 201/00*** *(2006.01)*
***C09K 5/10*** *(2006.01)*

(86) International application number:
**PCT/JP2016/063595**

(87) International publication number:
**WO 2016/178426 (10.11.2016 Gazette 2016/45)**

(54) **AGENT HAVING ANTI-ICE NUCLEATION ACTIVITY**

MITTEL MIT ANTI-ICE-NUKLEIERUNGSAKTIVITÄT

AGENT AYANT UNE ACTIVITÉ D'ANTI-NUCLÉATION DE LA GLACE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.05.2015 JP 2015095251**

(43) Date of publication of application:
**14.03.2018 Bulletin 2018/11**

(73) Proprietors:
- **The School Corporation Kansai University Suita-shi, Osaka 564-8680 (JP)**
- **Shin Nippon Yakugyo Co., Ltd. Tokyo 103-0001 (JP)**

(72) Inventors:
- **KAWAHARA, Hidehisa Suita-shi Osaka 564-8680 (JP)**
- **HIRANO, Yoshiaki Suita-shi Osaka 564-8680 (JP)**

(74) Representative: **Arends, William Gerrit Marks & Clerk LLP 15 Fetter Lane London EC4A 1BW (GB)**

(56) References cited:
**WO-A1-2008/033100**
**WO-A1-2010/143539**
**WO-A1-2011/136377**
**WO-A1-2012/023486**
**WO-A1-2012/121172**
**WO-A2-01/42388**
**JP-A- H04 505 329**
**JP-A- 2003 250 572**
**JP-A- 2015 038 170**

- **CLEMENS SORG ET AL: "Stufenweise Synthese von Oligotyrosinpeptiden", LIEBIGS ANN. CHEM., vol. 734, 1 January 1970 (1970-01-01), pages 180-186, XP055069786, DOI: 10.1002/jlac.19707340119**
- **ANNE BEILVERT ET AL: "Synthesis and evaluation of a tri-tyrosine decorated dextran MR contrast agent for vulnerable plaque detection", CHEMICAL COMMUNICATIONS, vol. 47, no. 19, 1 May 2011 (2011-05-01), pages 5506-5508, XP055500315, ISSN: 1359-7345, DOI: 10.1039/c1cc10849b**
- **ERI TAGAWA ET AL: "Anti-Ice Nucleation Activities of Tyrosine Peptide", BIOCONTROL SCIENCE, vol. 23, no. 2, 1 January 2018 (2018-01-01), pages 81-83, XP055500303, JP ISSN: 1342-4815, DOI: 10.4265/bio.23.81**

EP 3 293 240 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to an anti-ice nucleation activator, an antifreeze liquid, and a coating solution for preventing frost damage. The present invention further relates to a method for improving the anti-ice nucleation activity of a biological material, and a method for preserving a biological material.

### Background Art

Since foreign substances contained in water form ice nuclei, water solidifies at 0°C. Such foreign substances are called ice nucleation active substances. Typical examples of known ice nucleation active substances include bacteria of the *Pseudomonas* genus, silver iodide, and the like. In contrast, pure water contains no foreign substances; therefore, ice nucleation does not occur. Even if pure water is cooled to a temperature lower than the freezing point (0°C), for example, -39°C, pure water may not solidify (solidification). This is generally called "supercooling phenomenon."

Some anti-ice nucleation activators (supercooling accelerators), which promote the supercooling phenomenon, have previously been reported. The anti-ice nucleation activators are capable of forming water that does not freeze even at a temperature under the freezing point. As a result, expansion upon freezing does not occur, which allows cells of plants or animals to be preserved without being destroyed. Even if it is frozen once, only fine ice nuclei are formed, resulting in the occurrence of fine ice crystal formation. Thus, the application of anti-ice nucleation activators to the fields of food, biomaterials (organ preservation), etc., is expected.

For example, low-molecular-weight compounds, such as eugenol, which is a component of a spice (see Non-Patent Literature (NPL) 1); as well as high-molecular-weight compounds, such as polysaccharide from *Bacillus thuringiensis* (see NPL 2), have been reported to exhibit anti-ice nucleation activity.

However, although these anti-ice nucleation activators exhibit anti-ice nucleation activity towards bacteria, such as *Pseudomonas fluorescens*, which is an ice nucleation active substance, they exhibit low anti-ice nucleation activity towards silver iodide. Moreover, due to safety issues, the use of the anti-ice nucleation activators was difficult in the fields of food, biomaterials, etc.

### Citation List

#### Non-patent Literature

NPL 1: H. Kawahara et al., J. Antibact. Antifung. Agents, 1996, Vol. 24, pp. 95-100
NPL 2: Y. Yamashita et al., Biosci. Biotech. Biochem., 2002, Vol. 66, pp. 948-954

### Summary of Invention

#### Technical Problem

An object of the present invention is to provide an anti-ice nucleation activator, an antifreeze liquid, and a coating solution for preventing frost damage, that exhibit anti-ice nucleation activity widely towards ice nucleation active substances; and that are applicable to the fields of food, biological materials, environment, etc. Another object of the present invention is to provide a method for improving the anti-ice nucleation activity of a biological material, and a method for preserving a biological material.

#### Solution to Problem

In order to achieve the above objects, the present inventors conducted extensive research, and found that a tyrosine peptide, as well as a complex composed of one or more tyrosine peptides and a polymer, are capable of achieving the above objects. The inventors conducted further research based on these findings. The present invention has thus been accomplished.

More specifically, the present invention provides the following anti-ice nucleation activator, antifreeze liquid, coating solution for preventing frost damage, and the like.

Item 1. An anti-ice nucleation activator comprising:

a tyrosine peptide represented by Formula (1):

$$(X)_p\text{-}[Tyr]_n\text{-}(Y)_q \qquad (1)$$

wherein X and Y are identical or different, and each represents an amino acid residue,
n represents an integer of 2 to 5,
p represents an integer of 0 to 4, and
q represents an integer of 0 to 4,
with the proviso that p + q does not exceed 4,
wherein when p represents an integer of 2 to 4, two to four amino acid residues represented by $(X)_p$ may be identical or different, and when q represents an integer of 2 to 4, two to four amino acid residues represented by $(Y)_q$ may be identical or different; or
a complex composed of one or more of the tyrosine peptides and a polymer, wherein the one or more of the tyrosine peptides are bonded to the polymer.

Item 2. The anti-ice nucleation activator according to Item 1, wherein n represents an integer of 2 to 4.
Item 3. The anti-ice nucleation activator according to Item 1 or 2, wherein n represents 2 or 3, p represents 0 or 1, and q represents 0 or 1.
Item 4. An antifreeze liquid comprising the anti-ice nucleation activator of any one of Items 1 to 3, and water.
Item 5. The antifreeze liquid according to Item 4, comprising the anti-ice nucleation activator in an amount of 0.1 to 10 mg/mL.
Item 6. A coating solution for preventing frost damage comprising the complex of Item 1.
Item 7. A method for improving the anti-ice nucleation activity of a biological material, the method comprising the step of bringing the anti-ice nucleation activator of any one of Items 1 to 3 into contact with a biological material.
Item 8. A method for improving the anti-ice nucleation activity of food, the method comprising the step of bringing the anti-ice nucleation activator of any one of Items 1 to 3 into contact with food.
Item 9. A method for preserving a biological material, the method comprising the step of bringing the anti-ice nucleation activator of any one of Items 1 to 3 into contact with a biological material.
Item 10. A method for preserving food, the method comprising the step of bringing the anti-ice nucleation activator of any one of Items 1 to 3 into contact with food.
Item 11. Use of the tyrosine peptide or complex of Item 1 as an anti-ice nucleation activator.

Advantageous Effects of Invention

The present invention provides an anti-ice nucleation activator, antifreeze liquid, and coating solution for preventing frost damage that exhibit anti-ice nucleation activity widely towards ice nucleation active substances, and that are applicable to the fields of food, biological materials, environment, etc. The use of the anti-ice nucleation activator of the present invention is expected to enable long-term, low-temperature preservation of food, biological materials, etc.

Brief Description of Drawings

Fig. 1 is a photograph with regard to the droplet-freezing method of Vali.
Fig. 2 is a graph showing the effect of the polymerization degree of tyrosine (Examples 1 to 4 and Comparative Example 1).
Fig. 3 is a graph showing the effect of the concentration of a tyrosine trimer (Example 2 and 5 to 10).
Fig. 4 is graphs showing the anti-ice nucleation activity of a tri-peptide and a mono-amino acid (Example 2 and Comparative Examples 2 to 8).
Fig. 5 is a graph showing the effect on a different kind of ice nucleation active substance.
Fig. 6 shows the state of tofu after thawing (photographs), and the breaking loads (N) of tofu (Example 1 and Comparative Example 9) .
Fig. 7 is SEM photographs (magnification: 100x) of tofu that was cryopreserved for one month, followed by slow thawing or fast thawing (Examples 1 and 2 and Comparative Example 9).
Fig. 8 is SEM photographs (magnification: 100x) showing the state of frozen tofu (Example 2 and Comparative Example 9).
Fig. 9 is a graph showing the relationship (cooling curve) between the temperature of tofu and the cooling time

(Examples 1 and 2 and Comparative Example 9).

Fig. 10 is graphs showing the results of X-ray-photoelectron spectroscopy analysis. The graphs show the results of, from the top, P4VP-GGGYYY (Mw: 36000, 46000, 54000), GGGYYY, and piranha treatment only.

Fig. 11 is a graph showing the anti-ice nucleation activity of a tri-peptide, and a complex of a hexa-peptide with a polymer, in water (Comparative Example 10 and Examples 2 and 11).

Fig. 12 is a graph showing the anti-ice nucleation activity of a polymer and a complex of a tri-peptide or a hexa-peptide with a polymer on a glass surface (Comparative Example 11 and Examples 12 and 13).

Description of Embodiments

1. Anti-Ice Nucleation Activator

The anti-ice nucleation activator of the present invention comprises a tyrosine peptide, or a complex composed of one or more of the tyrosine peptides and a polymer, wherein the one or more of the tyrosine peptides are bound to the polymer. In this specification, the term "comprise/contain" encompasses the concepts of "comprise," "contain," "consist essentially of," and "consist of."

Tyrosine Peptide

A tyrosine peptide is a compound that has a tyrosine-tyrosine (Tyr-Tyr) structure. The tyrosine peptide refers to a compound of di- to pentatyrosine peptide. Such a tyrosine peptide is represented by Formula (1) :

$$(X)_p\text{-}[Tyr]_n\text{-}(Y)_q \qquad (1)$$

wherein X, Y, n, p, and q are as defined above.

The [Tyr] refers to a tyrosine residue ([$H_2N$-CH(-$CH_2C_6H_4$-4-OH)-COOH], [$H_2N$-CH(-$CH_2C_6H_4$-4-OH)-CO], [HN-CH(-$CH_2C_6H_4$-4-OH)-COOH], or [HN-CH(-$CH_2C_6H_4$-4-OH)-CO]), and the tyrosine in these tyrosine residues may be, for example, L-tyrosine or D-tyrosine. The tyrosine is preferably L-tyrosine, which is a naturally occurring amino acid.

n represents an integer of 2 to 5, more preferably an integer of 2 to 4, and particular preferably an integer of 2 or 3.

$[Tyr]_2$, in which n is 2, represents a structure [Tyr-Tyr], i.e., a structure [$H_2N$-CH(-$CH_2C_6H_4$-4-OH)-CO-NH-CH(-$CH_2C_6H_4$-4-OH)-COOH], [$H_2N$-CH(-$CH_2C_6H_4$-4-OH)-CO-NH-CH(-$CH_2C_6H_4$-4-OH)-CO], [HN-CH(-$CH_2C_6H_4$-4-OH)-CO-NH-CH(-$CH_2C_6H_4$-4-OH)-COOH], or [HN-CH(-$CH_2C_6H_4$-4-OH)-CO-NH-CH(-$CH_2C_6H_4$-4-OH)-CO].

$[Tyr]_3$, in which n is 3, represents a structure [Tyr-Tyr-Tyr], i.e., a structure [$H_2N$-CH(-$CH_2C_6H_4$-4-OH)-CO-NH-CH(-$CH_2C_6H_4$-4-OH)-CO-NH-CH(-$CH_2C_6H_4$-4-OH)-COOH], [$H_2N$-CH(-$CH_2C_6H_4$-4-OH)-CO-NH-CH(-$CH_2C_6H_4$-4-OH)-CO-NH-CH(-$CH_2C_6H_4$-4-OH)-CO], [HN-CH(-$CH_2C_6H_4$-4-OH)-CO-NH-CH(-$CH_2C_6H_4$-4-OH)-CO-NH-CH(-$CH_2C_6H_4$-4-OH)-COOH], or [HN-CH(-$CH_2C_6H_4$-4-OH)-CO-NH-CH(-$CH_2C_6H_4$-4-OH)-CO-NH-CH(-$CH_2C_6H_4$-4-OH)-CO].

$[Tyr]_4$, in which n is 4, represents a structure [Tyr-Tyr-Tyr-Tyr], i.e., a structure [$H_2N$-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-COOH], [$H_2N$-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO], [HN-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-COOH], or [HN-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO].

$[Tyr]_5$, in which n is 5, represents a structure [Tyr-Tyr-Tyr-Tyr-Tyr], i.e., a structure [$H_2N$-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-COOH], [$H_2N$-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO], [HN-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-COOH], or [HN-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO-NH-CH(-$CH_2C_6H_4$-4-OH)-CO].

p represents an integer of 0 to 4, and q represents an integer of 0 to 4, with the proviso that p + q does not exceed 4. When p represents an integer of 2 to 4, the two to four amino acid residues represented by $(X)_p$ may be identical or different. When q is an integer of 2 to 4, the two to four amino acid residues represented by $(Y)_q$ may be identical or different.

When p is 1, the amino acid residue represented by $(X)_p$ refers to a group ($H_2N$-CHR-CO) in which the hydroxyl group (OH) has been removed from the C-terminus of an amino acid. Here, R represents the side chain of the amino acid. When p is an integer of 2 to 4, the amino acid residues refer to a group ($H_2N$-CHR-CO) in which the hydroxyl group (OH) has been removed from the C-terminus of an amino acid, or a group (HN-CHR-CO) in which the hydroxyl group

(OH) has been removed from the C-terminus of an amino acid while the hydrogen atom has been removed from the N-terminus.

More specifically, $(X)_2$-, in which p is 2, represents the structure (X-X)-, i.e., the structure (amino acid residue-amino acid residue)-. For example, when X is Ser (serine), (Ser-Ser)- represents [$H_2N$-CH(-$CH_2OH$)-CO-NH-CH(-$CH_2OH$)-CO]-.

$(X)_3$-, in which p is 3, represents the structure (X-X-X)-. For example, when X is Ser (serine), (Ser-Ser-Ser)-represents [$H_2N$-CH(-$CH_2OH$)-CO-NH-CH(-$CH_2OH$)-CO-NH-CH(-$CH_2OH$)-CO]-.

$(X)_4$-, in which p is 4, represents the structure (X-X-X-X)-. For example, when X is Ser (serine), (Ser-Ser-Ser-Ser)-represents [$H_2N$-CH(-$CH_2OH$)-CO-NH-CH(-$CH_2OH$)-CO-NH-CH(-$CH_2OH$)-CO-NH-CH(-$CH_2OH$)-CO]-.

When q is 1, the amino acid residue represented by $(Y)_q$ refers to a group (HN-CHR-COOH) in which the hydrogen atom (H) has been removed from the N-terminus of an amino acid. Here, R represents the side chain of the amino acid. When q is 2 to 4, the amino acid residues refer to a group (HN-CHR-COOH) in which the hydrogen atom (H) has been removed from the N-terminus of an amino acid, or a group (HN-CHR-CO) in which the hydroxyl group (OH) has been removed from the C-terminus of an amino acid while the hydrogen atom has been removed from the N-terminus.

More specifically, -$(Y)_2$, in which q is 2, represents the structure -(Y-Y), i.e., the structure -(amino acid residue-amino acid residue). For example, when Y is Thr (threonine), -(Thr-Thr) represents -[HN-CH(-CH($CH_3$)OH)-CO-NH-CH(-CH($CH_3$)OH)-COOH].

-$(Y)_3$, in which q is 3, represents the structure -(Y-Y-Y). For example, when Y is Thr (threonine), -(Thr-Thr-Thr) represents -[HN-CH(-CH($CH_3$)OH)-CO-HN-CH(-CH($CH_3$)OH)-CO-NH-CH(-CH($CH_3$)OH)-COOH].

-$(Y)_4$, in which q is 4, represents the structure -(Y-Y-Y-Y). For example, when Y is Thr (threonine), -(Thr-Thr-Thr-Thr) represents -[HN-CH(-CH($CH_3$)OH)-CO-HN-CH(-CH($CH_3$)OH)-CO-HN-CH(-CH($CH_3$)OH)-CO-NH-CH(-CH($CH_3$)OH)-COOH].

Examples of amino acids in the "amino acid residues" include naturally occurring amino acids and non-naturally occurring amino acids. That is, the amino acid in an amino acid residue may be an L-amino acid, a D-amino acid, or a mixture thereof. The types of amino acids are not particularly limited as long as it is a compound that contains an amino group and a carboxyl group. Examples include $\alpha$-amino acids, $\beta$-amino acids, $\gamma$-amino acids, and $\delta$-amino acids, with naturally occurring $\alpha$-amino acids being preferable, and $\alpha$-amino acids that contain a hydroxyl group in the amino acid side chain being more preferable.

Examples of naturally occurring amino acids include glycine, L-alanine, L-valine, L-leucine, L-isoleucine, L-serine, L-threonine, L-aspartic acid, L-glutamine acid, L-asparagine, L-glutamine, L-lysine, L-arginine, L-cystine, L-methionine, L-phenylalanine, L-tyrosine, L-proline, L-tryptophan, L-histidine, and L-proline. Of these, examples of preferable naturally occurring amino acids include compounds that contain a hydroxyl group in the amino acid side chain, such as L-serine, L-threonine, and L-tyrosine, with L-tyrosine being more preferable.

Non-naturally occurring amino acids refer to all amino acids other than the above 20 types of naturally occurring amino acids, which constitute naturally occurring proteins. Specific examples include (1) a non-naturally occurring amino acid in which an atom of a naturally occurring amino acid is replaced with another substance, (2) an optical isomer or a regioisomer of the side chain of a naturally occurring amino acid, (3) a non-naturally occurring amino acid in which a substituent is introduced to the side chain of a naturally occurring amino acid, and (4) a non-naturally occurring amino acid in which the side chain of a naturally occurring amino acid is substituted to allow modification of the hydrophobicity, reactivity, a charge state, molecular size, an ability to form a hydrogen bond, etc.

In this specification, the tyrosine peptide or the amino acid may be a tyrosine peptide derivative or an amino acid derivative in which the backbone or side chain of a tyrosine peptide or amino acid is chemically or biologically (enzymatically) modified. Examples of modifications include, but are not limited to, functional group introduction (transformations), such as alkylation, acylation (more specifically, acetylation), hydroxylation, esterification, halogenation, amination, and amidation. Specific tyrosine peptide derivatives are described later.

The abbreviations for amino acids as used in this specification are in accordance with the rules of the IUPAC-IUB (IUPAC-IUB Communication on Biological Nomenclature, Eur. J. Biochem., 138, 9 (1984)) and "Guidelines for the preparation of specifications which contain nucleotide and/or amino acid sequence" (Japanese Patent Office), and those conventionally used in the related field. For amino acids and the like that may have optical isomers, L-form is referred to unless otherwise specified.

As used in this specification, "amino acids" that are present in various amino acid sequences mentioned in this specification are specified by well-known three-letter or one-letter abbreviations (see Table 1).

Table 1

| Codes | | Amino acids | Codes | | Amino acids |
|---|---|---|---|---|---|
| Three letters | One letter | | Three letters | One letter | |
| Ala | A | Alanine | Met | M | Methionine |
| Gly | G | Glycine | Leu | L | Leucine |
| Asp | D | Aspartic acid | Ile | I | Isoleucine |
| Glu | E | Glutamic acid | Tyr | Y | Tyrosine |
| Asn | N | Asparagine | Phe | F | Phenylalanine |
| Gln | Q | Glutamine | His | H | Histidine |
| Ser | S | Serine | Lys | K | Lysine |
| Thr | T | Threonine | Arg | R | Arginine |
| Pro | P | Proline | Trp | W | Tryptophan |
| Val | V | Valine | Cys | C | Cysteine |

As described above, in this specification, Tyr-Tyr is a compound [$H_2N-CH(-CH_2C_6H_4)-CO-NH-CH(-CH_2C_6H_4)-COOH$], in which tyrosines are condensed to form a peptide bond between the carboxylic acid of one tyrosine and the amino acid of another tyrosine.

A dimer of tyrosine (hereinafter sometimes referred to as a "tyrosine dimer") is not only a peptide consisting of the amino acid sequence Tyr-Tyr, but also a compound in which a further amino acid residue or amino acid residues are bound to one end or both ends of Tyr-Tyr. In this specification, a tyrosine dimer compound encompasses a tyrosine dimer, and a compound in which an amino acid residue or amino acid residues are bound to a tyrosine dimer.

Examples of tyrosine dimer compounds include compounds represented by Formula (2):

$$(X)_p\text{-[Tyr-Tyr]-}(Y)_q \qquad (2)$$

wherein X and Y are identical or different, and each represents an amino acid residue, p represents an integer of 0 to 4, and q represents an integer of 0 to 4, with the proviso that p + q does not exceed 4. When p is 2 to 4, two to four amino acid residues represented by $(X)_p$ may be identical or different, and when q is 2 to 4, two to four amino acid residues represented by $(Y)_q$ may be identical or different.

Specific examples of tyrosine dimer compounds include
Tyr-Tyr,
Ser-Tyr-Tyr,
Tyr-Tyr-Ser,
Ser-Tyr-Tyr-Ser,
Phe-Tyr-Tyr,
Tyr-Tyr-Phe,
Phe-Tyr-Tyr-Phe,
Thr-Tyr-Tyr,
Tyr-Tyr-Thr,
Thr-Tyr-Tyr-Thr,
Phe-Ser-Tyr-Tyr,
Ser-Tyr-Tyr-Phe,
Tyr-Tyr-Ser-Phe,
Tyr-Tyr-Ser-Tyr-Tyr,

Phe-Ser-Tyr-Tyr-Ser-Phe, and the like. Of these, preferable examples of tyrosine dimer compounds include Tyr-Tyr and a tyrosine dimer compound in which a hydroxyl-containing amino acid residue is bound to a tyrosine dimer, with Tyr-Tyr, Ser-Tyr-Tyr, Tyr-Tyr-Ser, Thr-Tyr-Tyr, and Tyr-Tyr-Thr being more preferable.

Examples of tyrosine peptides include tyrosine trimer compounds, tyrosine tetramer compounds, tyrosine pentamer compounds, and tyrosine hexamer compounds, in addition to the tyrosine dimer compounds described above.

The tyrosine trimer compound encompasses, in addition to Tyr-Tyr-Tyr, a compound in which a further amino acid residue or amino acid residues are bound to both ends or one end of Tyr-Tyr-Tyr. In this specification, a tyrosine trimer compound encompasses a tyrosine trimer and a compound in which an amino acid residue or amino acid residues

are bound to a tyrosine trimer.

Examples of tyrosine trimer compounds include compounds represented by Formula (3):

$$(X)_p\text{-[Tyr-Tyr-Tyr]-}(Y)_q \qquad (3)$$

wherein X and Y are identical or different, and each represents an amino acid residue, p represents an integer of 0 to 3, and q represents an integer of 0 to 3, with the proviso that p + q does not exceed 3. When p is 2 or 3, two or three amino acid residues represented by $(X)_p$ may be identical or different. When q is 2 or 3, two or three amino acid residues represented by $(Y)_q$ may be identical or different.

Specific examples of tyrosine trimer compounds include
Tyr-Tyr-Tyr,
Ser-Tyr-Tyr-Tyr,
Tyr-Tyr-Tyr-Ser,
Ser-Tyr-Tyr-Tyr-Ser,
Phe-Tyr-Tyr-Tyr,
Tyr-Tyr-Tyr-Phe,
Phe-Tyr-Tyr-Tyr-Phe,
Thr-Tyr-Tyr-Tyr,
Tyr-Tyr-Tyr-Thr,
Thr-Tyr-Tyr-Tyr-Thr,
Phe-Ser-Tyr-Tyr-Tyr,
Ser-Tyr-Tyr-Tyr-Phe,
Tyr-Tyr-Tyr-Ser-Phe,
Phe-Ser-Tyr-Tyr-Tyr-Ser-Phe, and the like. Of these, preferable examples of tyrosine trimer compounds include Tyr-Tyr-Tyr and a tyrosine trimer compound in which a hydroxyl-containing amino acid residue is bound to a tyrosine trimer, with Tyr-Tyr-Tyr being more preferable.

The tyrosine tetramer compound encompasses, in addition to Tyr-Tyr-Tyr-Tyr, a compound in which a further amino acid residue or amino acid residues are bound to both ends or one end of Tyr-Tyr-Tyr-Tyr. In this specification, the tyrosine tetramer compound encompasses a tyrosine tetramer and a compound in which an amino acid residue or amino acid residues are bound to a tyrosine tetramer.

Examples of tyrosine tetramer compounds include compounds represented by Formula (4):

$$(X)_p\text{-[Tyr-Tyr-Tyr-Tyr]-}(Y)_q \qquad (4)$$

wherein X and Y are identical or different, and each represents an amino acid residue, p represents an integer of 0 to 2, and q represents an integer of 0 to 2, with the proviso that p + q does not exceed 2. When p is 2, two amino acid residues represented by $(X)_p$ may be identical or different. When q is 2, two amino acid residues represented by $(Y)_q$ may be identical or different.

Specific examples of tyrosine tetramer compounds include
Tyr-Tyr-Tyr-Tyr,
Ser-Tyr-Tyr-Tyr-Tyr,
Tyr-Tyr-Tyr-Tyr-Ser,
Ser-Tyr-Tyr-Tyr-Tyr-Ser,
Phe-Tyr-Tyr-Tyr-Tyr,
Tyr-Tyr-Tyr-Tyr-Phe,
Phe-Tyr-Tyr-Tyr-Tyr-Phe,
Thr-Tyr-Tyr-Tyr-Tyr,
Tyr-Tyr-Tyr-Tyr-Thr,
Thr-Tyr-Tyr-Tyr-Tyr-Thr,
Phe-Ser-Tyr-Tyr-Tyr-Tyr,
Ser-Tyr-Tyr-Tyr-Tyr-Phe,
Tyr-Tyr-Tyr-Tyr-Ser-Phe,
Phe-Ser-Tyr-Tyr-Tyr-Tyr-Ser-Phe, and the like. Of these, preferable examples of tyrosine tetramer compounds include Tyr-Tyr-Tyr-Tyr.

The tyrosine pentamer compound encompasses, in addition to Tyr-Tyr-Tyr-Tyr-Tyr, a compound in which a further amino acid residue or amino acid residues are bound to both ends or one end of Tyr-Tyr-Tyr-Tyr-Tyr. In this specification, the tyrosine pentamer compound encompasses a tyrosine pentamer and a compound in which an amino acid residue or amino acid residues are bound to a tyrosine pentamer.

Examples of tyrosine pentamer compounds include compounds represented by Formula (5):

$$(X)_p\text{-[Tyr-Tyr-Tyr-Tyr-Tyr]-}(Y)_q \qquad (5)$$

wherein X and Y are identical or different, and each represents an amino acid residue, p represents an integer of 0 to 1, and q represents an integer of 0 to 1, with the proviso that p + q does not exceed 1.

Specific examples of tyrosine pentamer compounds include
Tyr-Tyr-Tyr-Tyr-Tyr,
Ser-Tyr-Tyr-Tyr-Tyr-Tyr,
Tyr-Tyr-Tyr-Tyr-Tyr-Ser,
Ser-Tyr-Tyr-Tyr-Tyr-Tyr-Ser,
Phe-Tyr-Tyr-Tyr-Tyr-Tyr,
Tyr-Tyr-Tyr-Tyr-Tyr-Phe,
Phe-Tyr-Tyr-Tyr-Tyr-Tyr-Phe,
Thr-Tyr-Tyr-Tyr-Tyr-Tyr,
Tyr-Tyr-Tyr-Tyr-Tyr-Thr,
Thr-Tyr-Tyr-Tyr-Tyr-Tyr-Thr,
Phe-Ser-Tyr-Tyr-Tyr-Tyr-Tyr,
Ser-Tyr-Tyr-Tyr-Tyr-Tyr-Phe,
Tyr-Tyr-Tyr-Tyr-Tyr-Ser-Phe,
Phe-Ser-Tyr-Tyr-Tyr-Tyr-Tyr-Ser-Phe, and the like. Of these, preferable examples of tyrosine pentamer compounds include Tyr-Tyr-Tyr-Tyr-Tyr.

Of these, a tyrosine peptide in which n is 2 to 4 is preferable, a tyrosine peptide in which n is 2 or 3 is more preferable, a tyrosine peptide in which n is 2 or 3, p is 0 or 1, and q is 0 or 1 is still more preferable, and a tyrosine peptide (a tyrosine trimer, Tyr-Tyr-Tyr) in which n is 3, p is 0, and q is 0 is particularly preferable.

In this specification, the tyrosine peptide encompasses the tyrosine dimer compounds, tyrosine trimer compounds, tyrosine tetramer compounds, and tyrosine pentamer compounds mentioned above, as well as compounds that have a chemical structure similar to these compounds (the tyrosine peptide derivatives mentioned above). Examples of tyrosine peptide derivatives include compounds in which hydrogen of a tyrosine peptide (e.g., the hydrogen atom of an amino group; the hydrogen atom of the hydroxyl group in the tyrosine side chain) is functionalized, i.e., alkylated, acylated (more specifically, acetylated etc.), esterified, halogenated, or amidated; regioisomers with hydroxy at a different position (m-tyrosine, o-tyrosine); and the like. Of these, preferable tyrosine peptide derivatives are a compound in which hydrogen of tyrosine peptide is replaced by $C_{1-6}$ alkyl; a compound in which hydrogen of tyrosine peptide is replaced by $C_{3-8}$ cycloalkyl; and a compound in which hydrogen of tyrosine peptide is replaced by aryl.

Examples of "alkyl" include, but are not particularly limited to, $C_{1-6}$ linear alkyl and $C_{3-6}$ branched alkyl groups. Specific examples include methyl, ethyl, n-propyl, isopropyl, n-butyl, isobutyl, s-butyl, t-butyl, n-pentyl, 2-methylbutyl, 1-methylbutyl, neopentyl, 1,2-dimethylpropyl, 1,1-dimethylpropyl, n-hexyl, 4-methylpentyl, 3-methylpentyl, 2-methylpentyl, 1-methylpentyl, 3,3-dimethylbutyl, 2,3-dimethylbutyl, 1,3-dimethylbutyl, 2,2-dimethylbutyl, 1,2-dimethylbutyl, 1,1-dimethylbutyl, 2-ethylbutyl, 1-ethylbutyl, 1,1,2-trimethylpropyl, 1-ethyl-2-methylpropyl, and the like. The alkyl is preferably $C_{1-6}$ linear alkyl, and more preferably methyl, ethyl, and n-butyl. The alkyl may have 1 to 6 substituents, such as halogen (e.g., fluorine, chlorine, bromine, and iodine), cyano, nitro, cycloalkyl (e.g., cyclopropyl, cyclobutyl, cyclopentyl, and cyclohexyl), and aryl (e.g., phenyl and naphthyl).

In this specification, "n-" means normal, "s-" means secondary (sec-), and "t-" means tertiary (tert-).

Examples of "cycloalkyl" include, but are not particularly limited to, $C_{3-8}$ cycloalkyl groups. Specific examples include cyclopropyl, cyclobutyl, cyclopentyl, cyclohexyl, cycloheptyl, cyclooctyl, and the like. The cycloalkyl is preferably $C_{3-7}$ cycloalkyl, more preferably $C_{5-7}$ cycloalkyl, and particularly preferably cyclohexyl. The cycloalkyl may have 1 to 6 substituents, such as halogen (e.g., fluorine, chlorine, bromine, and iodine), cyano, nitro, alkyl (e.g., $C_{1-6}$ alkyl), and aryl (e.g., phenyl and naphthyl).

Examples of "aryl" include, but are not particularly limited to, monocyclic aryl and aryl having two or more cyclic rings. Specific examples include phenyl, naphthyl, anthranyl, phenanthryl, and the like. The aryl is preferably monocyclic aryl or aryl having two cyclic rings, and more preferably phenyl. The aryl may have 1 to 6 substituents, such as halogen (e.g., fluorine, chlorine, bromine, and iodine), cyano, nitro, alkyl (e.g., $C_{1-6}$ alkyl), cycloalkyl (e.g., cyclopropyl, cyclobutyl, cyclopentyl, and cyclohexyl), and aryl (e.g., phenyl and naphthyl).

The tyrosine peptides may be used alone, or in a combination of two or more.

For the tyrosine peptides, commercially available products may be used. If there are no commercially available products, tyrosine peptides may be produced in accordance with a known production method. For example, a compound in which the amino group ($-NH_2$) of tyrosine is converted into dimethylamino ($-NMe_2$) may be produced by reacting adenine and a methylating agent (e.g., methyl iodide).

Complex Composed of Tyrosine Peptide and Polymer

The complex of the present invention is composed of one or more tyrosine peptides and a polymer, and the one or more of the tyrosine peptides are bound to this polymer. The tyrosine peptide may be bound to any of the backbone, a side chain, and an end of the polymer.

The polymer is not limited as long as the tyrosine peptide can bind. In particular, a compound containing a group to which a peptide can bind (e.g., a carboxyl group and an amino group) may be suitably used. Examples of polymers include polyethylene, polypropylene, polybutadiene, polytetrafluoroethylene, polyglycolic acid, polylactic acid, polyester, polyamide, polyethylene glycol, polyalkylene glycol, polyether, polyetheretherketone, polyethersulfone, polyurethane, polysulfone, polyamine, polyurea, polyimide, polyacrylic acid, polymethacrylic acid, polymethyl acrylate, polymethylmethacrylate, polyacrylonitrile, polystyrene, polyvinyl alcohol, polyvinyl chloride, polyvinylidene chloride, poly 4-vinylpyridine, cellulose, amylose, amylopectin, and the like. Of these, poly 4-vinylpyridine, polyacrylic acid, and polymethacrylic acid are preferable.

The average molecular weight of the polymer is, but not limited to, for example, within a range of 1,000 to 100,000. The average molecular weight as used herein may be either a number average molecular weight or a weight average molecular weight, and these average molecular weights can be measured by gel permeation chromatography (GPC) or the like.

The tyrosine peptide and the polymer may be arbitrarily bound to each other by using a known method. For example, a polymerization initiator, such as 4,4'-azobis(4-cyanovaleric acid) (ACVA), is first bound to a tyrosine peptide, followed by radical polymerization to synthesize a polymer. It is also possible to allow a tyrosine peptide to be bound to a polymer through an appropriate crosslinking agent. The crosslinking agent is not particularly limited as long as it is a divalent crosslinking agent, which is capable of binding a tyrosine peptide to a polymer. When the polymer contains a carboxyl group or an amino group, the polymer can bind to a tyrosine peptide by forming a peptide bond.

The tyrosine peptide to be bound to a polymer is not limited as long as it is a tyrosine peptide described above. In particular, it is preferable that a tyrosine peptide in which either X or Y represents 3 or 4 residues (i.e., p or q is 3 or 4) be bound to a polymer via the residues. This is because when X and Y function as a spacer, the tyrosine moiety is assumed to easily exert the anti-ice nucleation active performance. In this case, X and Y are not particularly limited, and are each preferably glycine.

When two or more tyrosine peptides are bound per molecule of the polymer, the tyrosine peptides may be of a single kind or a combination of two or more kinds. A single kind or a combination of two or more kinds of complexes may also be used.

The following describes the anti-ice nucleation activator of the present invention as a solid, unless otherwise specified; however, the anti-ice nucleation activator of the present invention is not limited to a solid. Specifically, the anti-ice nucleation activator of the present invention may be in the form of either a liquid or a solid. For example, if the anti-ice nucleation activator of the present invention is a solid, the solid may be used as an anti-ice nucleation activator by bringing the solid into contact with a biological material (e.g., food (e.g., edible seafood; plants, such as vegetables; edible meat, such as beef, pork, and chicken; protein-modified processed products, such as tofu and yogurt; and beverages) and biomaterials (e.g., cells (tissue) of plants or animals; human or animal blood; and human or animal organs or a portion thereof)) so as to be dissolved in moisture of the biological material. If the anti-ice nucleation activator of the present invention is a liquid, the liquid may be used as an anti-ice nucleation activator for the biological material above by bringing the liquid into contact with the biological material (e.g., by spraying or dropping).

The anti-ice nucleation activator of the present invention may further comprise other components. Examples of other components include, but are not particularly limited to, known anti-ice nucleation activators (e.g., the anti-ice nucleation activator disclosed in JP2010-121052A), water, ethanol, and the like.

When the anti-ice nucleation activator of the present invention is formulated, the dosage form is not particularly limited. Examples of the dosage form include solutions, suspensions, emulsions, tablets, capsules, granules, powders, creams, ointments, and the like.

2. Antifreeze Liquid

The antifreeze liquid of the present invention comprises the tyrosine peptide or the complex (anti-ice nucleation activator) composed of one or more of the tyrosine peptides and a polymer. An antifreeze liquid refers to a liquid that does not freeze at the freezing point (0°C) of ice.

The amount of the tyrosine peptide or the complex (anti-ice nucleation activator) contained in the antifreeze liquid is not particularly limited, and is usually within a range of 0.001 to 100 mg/mL, and preferably within a range of 0.1 to 10 mg/mL. The amount of the tyrosine peptide contained in the antifreeze liquid is not particularly limited, and is usually within a range of 0.001 to 100 mM, and preferably within a range of 0.1 to 10 mM.

The water may be pure water or tap water. The water may contain an ice nucleation active substance.

Examples of ice nucleation active substances include, but are not particularly limited to, *Pseudomonas* bacteria, silver iodide, fluoren-9-one, phenazine, metaldehyde, and the like. Of these, preferable ice nucleation active substances are *Pseudomonas syringae*, *Pseudomonas fluorescens*, and silver iodide.

A single kind or a combination of two or more kinds of ice nucleation active substances may be contained in the anti-ice nucleation activity composition.

The amount of the ice nucleation active substance contained in the antifreeze liquid is not particularly limited, and is usually within a range of 0.001 to 100 mg/mL, and preferably within a range of 0.1 to 10 mg/mL.

The weight ratio of the anti-ice nucleation activator to the ice nucleation active substance in the antifreeze liquid is usually 1:10 to 1:200, preferably 1:10 to 1:100, and more preferably 1:10 to 1:20.

When a biological material (e.g., food (e.g., edible seafood; plants, such as vegetables; edible meat, such as beef, pork, and chicken; protein-modified processed products, such as tofu and yogurt; and beverages) and biomaterials (e.g., cells (tissue) of plants or animals, and human or animal organs or a portion thereof)) is immersed in the antifreeze liquid of the present invention, or when the antifreeze liquid of the present invention is sprayed or dropped on the biological material, and cooling is performed, long-term, low-temperature preservation is possible usually at a temperature of 0°C or lower, in particular within a temperature range of about 0°C to -15°C, without the biological material being frozen (destroyed); or, even when it is frozen once, long-term, low-temperature preservation is possible since only fine ice nucleation is formed, which results in the occurrence of fine ice crystal formation.

## 3. Method for Improving the Anti-Ice Nucleation Activity of Biological Material

The method for improving the anti-ice nucleation activity of a biological material of the present invention comprises the step of bringing the above anti-ice nucleation activator (supercooling accelerator) or antifreeze liquid into contact with a biological material.

The biological materials are the same as the biological materials described above in sections 1 and 2.

Contact means that the anti-ice nucleation activator (supercooling accelerator), when it is a solid, is brought into contact with (e.g., uniformly sprinkled on) a biological material. The anti-ice nucleation activator is thereby dissolved in moisture of the biological material to exert the anti-ice nucleation activity. The anti-ice nucleation activator (supercooling accelerator) in a liquid form and the antifreeze liquid may be used as an anti-ice nucleation activator for the biological material by bringing the liquid into contact with (e.g., sprayed or dropped on) the biological material.

## 4. Method for Improving Anti-Ice Nucleation Activity of Food

The method for improving the anti-ice nucleation activity of food of the present invention comprises the step of bringing the anti-ice nucleation activator (supercooling accelerator) or antifreeze liquid into contact with food.

The food is the same as the food described above in sections 1 and 2. The meaning of contact is as described above in section 3.

## 5. Method for Preserving Biological Material

The method for preserving a biological material of the present invention comprises the step of bringing the anti-ice nucleation activator (supercooling accelerator) or antifreeze liquid into contact with a biological material.

The biological material is as described above in sections 1 and 2. The meaning of contact is as described above in section 3.

## 6. Method for Preserving Food

The method for preserving food of the present invention comprises the step of bringing the anti-ice nucleation activator (supercooling accelerator) or antifreeze liquid into contact with food.

The food is the same as the food described above in sections 1 and 2. The meaning of contact is as described above in section 3.

## 7. Application

The anti-ice nucleation activator (supercooling accelerator) or antifreeze liquid of the present invention is widely applicable to food fields (e.g., quality preservatives for food and beverages (food preservatives and beverage preservatives)); medical fields (e.g., cell preservatives, blood preservatives, and organ preservatives); cosmetic fields; environmental fields (e.g., coating compositions, agents for preventing frost damage, and frost adhesion inhibitors); and the like.

The anti-ice nucleation activator of the present invention may be added to a solution so as to be used as an

antifreeze liquid. This antifreeze liquid may be used as a food preservation solution, a beverage preservation solution, a cell preservation solution, an organ preservation solution, a solution for preventing frost damage, a solution for inhibiting frost adhesion, and the like.

Further, the complex (supercooling accelerator) composed of one or more tyrosine peptides and a polymer can be adsorption-immobilized on an object such as glass, metal, and resin via its polymer moiety, and is thus particularly effective as a component of a coating solution for preventing frost damage or for inhibiting frost adhesion.

Quality Preservative (Food Preservative and Beverage Preservative)

Examples of food and beverages for which the quality preservative (food preservative (food preservation solution) and beverage preservative (beverage preservation solution)) of the present invention can be used include, but are not limited to, perishable food, such as vegetables, fish, and meat (e.g., chicken, pork, and beef); beverages, such as juice; processed food, such as tofu and *Koya-tofu* (freeze-dried tofu); and the like.

The use of the quality preservative of the present invention enables preservation of food and beverages. When the food or beverages are imported, exported, or transported, it is possible to convert the cryopreservation into supercooling preservation, which makes it possible to reduce electric power etc.

Cell Preservative (Cell Preservation Solution)

The cells for which the cell preservative (cell preservation solution) of the present invention can be used are not particularly limited, as long as they are cells of plants or animals. Examples include human cells, sperm, ovum, and the like.

The use of the cell preservative (cell preservation solution) of the present invention enables preservation without destroying cells.

Blood Preservative (Blood Preservation Solution)

The blood for which the blood preservative (blood preservation solution) of the present invention can be used is not particularly limited, as long as it is the blood of a human or an animal (excluding a human). Examples include whole blood, plasma, serum, and the like. The blood preservative (blood preservation solution) of the present invention may also be used for blood constituents, such as white blood cells, red blood cells, plasma, and thrombocytes.

Organ Preservative (Organ Preservation Solution)

The organs for which the organ preservative (organ preservation solution) of the present invention can be used are not particularly limited, as long as they are organs of a human or an animal (excluding a human), or a portion thereof.

The organ preservative (organ preservation solution) of the present invention may be used as a preservation solution for an organ taken at the time of an organ transplantation; a preservation solution for long-term preservation of an organ; and the like.

Agent for Preventing Frost Damage (Solution for Preventing Frost Damage)

The agent for preventing frost damage (solution for preventing frost damage) of the present invention can be used as a computer or car engine coolant; a frost adhesion inhibitor for freezers, etc.; an antifog agent for car windows; an agent for preventing dew condensation in tunnels; and the like.

The anti-ice nucleation activator or antifreeze liquid of the present invention may be used after mixing with a coating composition or the like. The target such as metal or resin coated with a coating composition containing the anti-ice nucleation activator of the present invention is prevented from frost damage or frost adhesion.

Examples

The present invention is described below in detail with reference to Examples. However, the present invention is not limited to these Examples.

Measurement Device

In the Production Examples, MALDI-Tof mass spectrometry was performed on a KRATOS AXIMA-CFR (Shimadzu Corporation).

Starting Materials and Reagents

- Fmoc-Tyr(tBu)-Alko-PEG Resin: N-α-(9-Fluorenylmethoxycarbonyl)-O-(t-butyl)-L-tyrosine p-methoxybenzyl alcohol polyethyleneglycol resin (Watanabe Chemical Industries, Ltd.)

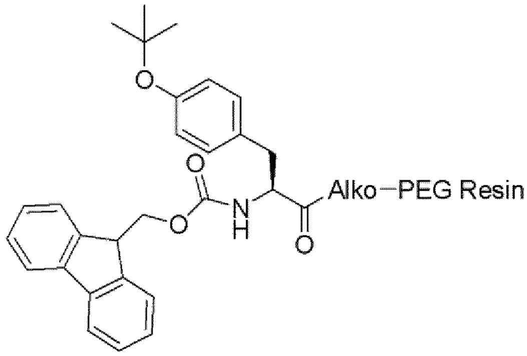

- Fmoc-Tyr(tBu)-OH: N-α-(9-Fluorenylmethoxycarbonyl)-O-(t-butyl)-L-tyrosine (Watanabe Chemical Industries, Ltd.)

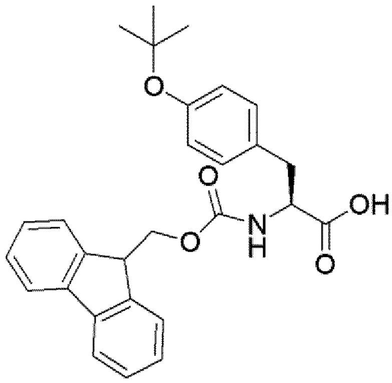

- DMSO: dimethylsulfoxide (Wako Pure Chemical Industries, Ltd.)
- DMF: dimethylformamide (Wako Pure Chemical Industries, Ltd.)
- PPD: piperidine (Wako Pure Chemical Industries, Ltd.)
- NMM: N-methylmorpholine (Wako Pure Chemical Industries, Ltd.)
- DMT-MM: 4-(4,6-Dimethoxy-1,3,5-triazin-2-yl)-4-methylmorpholinium chloride
- n-Hydrate (Wako Pure Chemical Industries, Ltd.)
- TFA: trifluoroacetic acid (Wako Pure Chemical Industries, Ltd.)

Production Example 1 (Tyrosine Dimer)

Fmoc-Tyr(tBu)-Alko-PEG Resin (0.917 g, 0.220 mmol) was swollen for 30 minutes with a 25% DMSO/DMF solution. Thereafter, a step of replacing the solvent in the resin with a large amount of DMF for 1 minute, and removing DMF (hereinafter referred to as "the replacing step") was repeated three times, and subsequently the Fmoc group was removed from the Fmoc-Tyr(tBu)-Alko-PEG Resin using a 20% PPD/DMF solution. Using the obtained Tyr(tBu)-Alko-PEG Resin, the replacing step that uses DMF for 1 minute was performed 3 times, and sequentially the replacing step that uses methanol for 1 minute was performed 3 times.

Subsequently, the obtained Tyr(tBu)-Alko-PEG Resin was swollen for 30 minutes with a 25% DMSO/DMF solution. Thereafter, using the Tyr(tBu)-Alko-PEG Resin, the replacing step that uses DMF for 1 minute was performed 3 times, and Fmoc-Tyr(tBu)-OH (3.0 eq), NMM (3.0 eq), and DMT-MM (3.0 eq) were added to this resin to perform a condensation reaction for 120 minutes. After the completion of the condensation reaction was confirmed by the sodium trinitrobenzene sulfonic acid (TNBS) method, the replacing step that uses DMF for 1 minute was performed 3 times, and sequentially the replacing step that uses methanol for 1 minute was performed 3 times to thus obtain Fmoc-Tyr(tBu)-Tyr(tBu)-Alko-PEG Resin.

TFA (9.5 mL) and ultra-pure water (Milli-Q (trademark) ultra-pure water purification system, Merck KGaA) (0.5 mL) were added to the obtained Fmoc-Tyr(tBu)-Tyr(tBu)-Alko-PEG Resin, and a deprotection reaction and a cleavage reaction were performed. Then, 50 mL of diethyl ether was added to the obtained reaction solution to obtain a tyrosine dimer-resin mixture (precipitate). After filtering off the precipitate, a large amount of TFA was added to the filtrate, and dissolved tyrosine dimer was collected, followed by vacuum concentration. After concentration, water was added to the residue to collect only the soluble portion, and the tyrosine dimer was fractionated by HPLC using a water-acetonitrile gradient (Tosoh Corporation, detector: UV (UV-8020), pump (DP-8020), autosampler (AS-8071), column (YMC Protein-RP 250 mm x 4.6 mm)) and dialyzed. After dialysis, lyophilization was performed to obtain a tyrosine dimer, i.e., the target product. The HPLC analysis confirmed that this compound exhibited a single peak.
Tyrosine dimer:
MALDI-TOF-MA: $[M+H]^+$ = 508.267, $[M+Na]^+$ = 530.306

Production Example 2 (Tyrosine Trimer)

The Fmoc-Tyr(tBu)-Tyr(tBu)-Alko-PEG Resin obtained in Production Example 1 was repeatedly subjected to the method described in Production Example 1, and Tyr(tBu)-Tyr(tBu)-Alko-PEG Resin and Fmoc-Tyr(tBu)-OH (3.0 eq) were subjected to a condensation reaction to obtain Fmoc-Tyr(tBu)-Tyr(tBu)-Tyr(tBu)-Alko-PEG Resin. Thereafter, a tyrosine trimer, i.e., the target product, was obtained in accordance with a purification method similar to that described in Production Example 1. The HPLC analysis confirmed that this compound exhibited a single peak. Tyrosine trimer:
MALDI-TOF-MA: $[M+H]^+$ = 508.267, $[M+Na]^+$ = 530.306, $[M+K]^+$ = 546.244

Production Example 3 (Tyrosine Tetramer)

The Fmoc-Tyr(tBu)-Tyr(tBu)-Tyr(tBu)-Alko-PEG Resin obtained in Production Example 2 was repeatedly subjected to the method described in Production Example 1, and Tyr(tBu)-Tyr(tBu)-Tyr(tBu)-Alko-PEG Resin and Fmoc-Tyr(tBu)-OH (3.0 eq) were subjected to a condensation reaction to obtain Fmoc-Tyr(tBu)-Tyr(tBu)-Tyr(tBu)-Tyr(tBu)-Alko-PEG Resin. Thereafter, the tyrosine tetramer (SEQ ID NO: 1), i.e., the target product, was obtained in accordance with a purification method similar to that described in Production Example 1. The HPLC analysis confirmed that this compound exhibited a single peak.
Tyrosine tetramer:
MALDI-TOF-MA: $[M+H]^+$ = 671.257, $[M+Na]^+$ = 693.713, $[M+K]^+$ = 709.701

Production Example 4 (Tyrosine Pentamer)

The Fmoc-Tyr(tBu)-Tyr(tBu)-Tyr(tBu)-Tyr(tBu)-Alko-PEG Resin obtained in Production Example 3 was repeatedly subjected to the method described in Production Example 1, and Tyr(tBu)-Tyr(tBu)-Tyr(tBu)-Tyr(tBu)-Alko-PEG Resin and Fmoc-Tyr(tBu)-OH (3.0 eq) were subjected to a condensation reaction to obtain Fmoc-Tyr(tBu)-Tyr(tBu)-Tyr(tBu)-Tyr(tBu)-Tyr(tBu)-Alko-PEG Resin. Thereafter, the tyrosine pentamer (SEQ ID NO: 2), i.e., the target product, was obtained in accordance with a purification method similar to that described in Production Example 1. The HPLC analysis confirmed that this compound exhibited a single peak.
Tyrosine pentamer:
MALDI-TOF-MA: $[M+H]^+$ = 834.893, $[M+Na]^+$ = 857.210, $[M+K]^+$ = 872.909

Example 1

An aqueous solution was prepared by dissolving the tyrosine dimer (Tyr-Tyr) obtained in Production Example 1 in ultra-pure water (Milli-Q (trademark) ultra-pure water purification system, Merck KGaA) to a concentration of 2.0 mM.

Examples 2 to 4

Each aqueous solution was produced in a manner similar to that described in Example 1, except that the anti-ice nucleation activators shown in Table 2 (the tyrosine peptides of Production Examples 2 to 4) were used in place of the tyrosine dimer.

Comparative Example 1

An aqueous solution was produced in a manner similar to that described in Example 1, except that tyrosine

(Tyr, Wako Pure Chemical Industries, Ltd.) was used in place of the tyrosine dimer.

Test Example 1: Evaluation of Anti-Ice Nucleation Active Performance (Supercooling Acceleration Performance)

The anti-ice nucleation active performance (supercooling acceleration performance) of the anti-ice nucleation activators comprising the tyrosine peptides (Examples 1 to 4) and the anti-ice nucleation activator comprising tyrosine (Comparative Example 1) as shown in Table 2 below was evaluated according to the following method.

Nine hundred microliters of an aqueous solution was prepared by dissolving silver iodide in ultra-pure water to a silver iodide concentration of 1.0 mg/mL.

Each sample for evaluation was prepared by mixing 100 μL of the aqueous solutions of Examples 1 to 4 and Comparative Example 1 with 900 μL of the aqueous solution in which silver iodide was dissolved. A blank sample was prepared by mixing 100 μL of ultra-pure water with 900 μL of the aqueous solution in which silver iodide was dissolved.

The anti-ice nucleation active performance (supercooling acceleration performance) was measured by using the droplet-freezing method of Vali. More specifically, in accordance with the droplet-freezing method of Vali (Fig. 1), an aluminum film was placed on the copper plate of a cold plate chiller (CoolAce CCA-1000, Tokyo Rikakikai Co., Ltd.), and the film was coated with a silicone oil suspension diluted with acetone and chloroform (a solution of acetone:chloroform = 1:2). Then, each sample for evaluation or the blank sample was dropped (10 μL each) on 30 points of the surface, the temperature was decreased at a rate of 1.0°C/min, and the temperature at which 50% of the 30 droplets were frozen was considered to be $T_{50}$.

The droplet-freezing temperature of each sample was referred to as Sample$T_{50}$, and the droplet-freezing temperature of the blank sample was referred to as Blank$T_{50}$. Then, the anti-ice nucleation activity level $\Delta T_{50}$ (°C) was calculated by the following formula:

$$\text{Formula: } \Delta T_{50}\ (^\circ C) = \text{Blank}T_{50} - \text{Sample}T_{50}$$

Table 2 below and Fig. 2 show the evaluation results of the anti-ice nucleation activators of Examples 1 to 4 and Comparative Example 1. The results are average values of the results of the test performed three times.

Table 2

| | Anti-ice nucleation activator comprising tyrosine peptide | Anti-ice nucleation activity level (°C) |
|---|---|---|
| Example 1 | Tyrosine dimer | 2.6 |
| Example 2 | Tyrosine trimer | 10.1 |
| Example 3 | Tyrosine tetramer | 1.8 |
| Example 4 | Tyrosine pentamer | 1.7 |
| Comparative Example 1 | Tyrosine | 0.3 |

Results

The anti-ice nucleation activity level of all of the anti-ice nucleation activators of Examples 1 to 4 was higher than 0, indicating that they exerted the anti-ice nucleation activity.

In contrast, the tyrosine of Comparative Example 1 showed no anti-ice nucleation activity.

Of the anti-ice nucleation activators of Examples 1 to 4, the tyrosine trimer (Example 2) achieved the highest anti-ice nucleation activity level, showing the most excellent anti-ice nucleation activity. Next, Test Example 2 was conducted to analyze the effect of concentration of tyrosine trimer.

Examples 5 to 10

Aqueous solutions were prepared by dissolving tyrosine trimer in ultra-pure water to a concentration of 0.1 mM (Example 5), 0.2 mM (Example 6), 0.5 mM (Example 7), 1.0 mM (Example 8), 5.0 mM (Example 9), and 10.0 mM (Example 10).

Test Example 2: Effect of Tyrosine Trimer Concentration

The effect of the concentration of the tyrosine trimer-containing anti-ice nucleation activator on the anti-ice nucleation activity was analyzed.

Nine hundred microliters of an aqueous solution was prepared by dissolving silver iodide in ultra-pure water to a silver iodide concentration of 1.0 mg/mL.

Each sample for evaluation was prepared by mixing 100 μL of the aqueous solutions of Examples 2 and 5 to 10 with 900 μL of the aqueous solution in which silver iodide was dissolved. A blank sample was prepared by mixing 100 μL of ultra-pure water with 900 μL of the aqueous solution in which silver iodide was dissolved.

The anti-ice nucleation active performance (supercooling acceleration performance) was measured by using the droplet-freezing method of Vali as described in Test Example 1. Table 3 below and Fig. 3 show the evaluation results regarding the anti-ice nucleation activators of Examples 2 and 5 to 10. The results are average values of the results of the test performed three times.

Table 3

| | Tyrosine trimer concentration (mM) | Anti-ice nucleation activity level (°C) |
|---|---|---|
| Example 5 | 0.1 | 2.3 |
| Example 6 | 0.2 | 3.7 |
| Example 7 | 0.5 | 1.8 |
| Example 8 | 1.0 | 5.7 |
| Example 2 | 2.0 | 10.1 |
| Example 9 | 5.0 | 9.2 |
| Example 10 | 10.0 | 9.2 |

Results

The tyrosine trimer exhibited anti-ice nucleation active performance at all of the concentration levels within the range of 0.1 to 10.0 mM (Examples 2 and 5 to 10). In particular, the anti-ice nucleation activity level at a tyrosine trimer concentration of 2.0 mM (1.0 mg/mL) (Example 2) was most excellent (10.1°C).

Test Example 3: Anti-Ice Nucleation Activity Test for Mono-Amino Acid or Tri-Peptide Other Than Tyrosine Trimer

The anti-ice nucleation active performance of mono-amino acid and tri-peptide other than tyrosine trimer was analyzed.

The anti-ice nucleation activity test was performed in a manner similar to the method described in Test Example 1, except that a phenylalanine trimer (Phe-Phe-Phe, Funakoshi Co., Ltd., Comparative Example 3), a serine trimer (Ser-Ser-Ser, Funakoshi Co., Ltd., Comparative Example 5), or a threonine trimer (Thr-Thr-Thr, Funakoshi Co., Ltd., Comparative Example 7) was used in place of the tyrosine trimer.

The anti-ice nucleation activity test was performed in a manner similar to the method described in Test Example 1, except that tyrosine (Tyr, Wako Pure Chemical Industries, Ltd., Comparative Example 2), phenylalanine (Phe, Wako Pure Chemical Industries, Ltd., Comparative Example 4), serine (Ser, Wako Pure Chemical Industries, Ltd., Comparative Example 6), or threonine (Thr, Wako Pure Chemical Industries, Ltd., Comparative Example 8) was used in place of the tyrosine trimer.

Table 4 below and Fig. 4 show the results. The results are average values of the results of the test performed three times.

Table 4

| | Anti-ice nucleation activator | Anti-ice nucleation activity level (°C) |
|---|---|---|
| Example 2 | Tyrosine trimer | 10.1 |
| Comparative Example 2 | Tyrosine | 0.3 |
| Comparative Example 3 | Phenylalanine trimer | 0.3 |

(continued)

| | Anti-ice nucleation activator | Anti-ice nucleation activity level (°C) |
|---|---|---|
| Comparative Example 4 | Phenylalanine | 0.5 |
| Comparative Example 5 | Serine trimer | 0.4 |
| Comparative Example 6 | Serine | 0.1 |
| Comparative Example 7 | Threonine trimer | -0.3 |
| Comparative Example 8 | Threonine | 0.1 |

Results

The tyrosine trimer (Example 2) achieved a significantly improved anti-ice nucleation activity level, compared to the tyrosine (Comparative Example 2).

Except for the tyrosine trimer, however, the phenylalanine trimer (Comparative Example 3), the serine trimer (Comparative Example 5), and the threonine trimer (Comparative Example 7) achieved no improvement in the anti-ice nucleation activity level, compared to the phenylalanine, serine, and threonine (Comparative Examples 4, 6, and 8).

Test Example 4: Effect on Different Kind of Ice Nucleation Active Substance

A sample for evaluation of Example 1 was prepared in a manner similar to that described in Test Example 1, except that *Pseudomonas fluorescens*, which is the Pseudomonas bacterium, was used in place of silver iodide, which is an ice nucleation active substance. The anti-ice nucleation active performance (supercooling acceleration performance) was measured by using a test method similar to that described in Test Example 1. According to the results, the tyrosine trimer-containing anti-ice nucleation activator also showed anti-ice nucleation active performance (1.9°C) towards *Pseudomonas fluorescens* as well, in addition to silver iodide. Fig. 5 shows the results. The results are average values of the results of the test performed three times.

Test Example 5: Anti-Ice Nucleation Activity Test With Respect to Biological Material (Beef Liver)

Ten microliters of an aqueous solution in which the aqueous solution of the anti-ice nucleation activator of Example 2 (tyrosine trimer) was diluted 10-fold with ultra-pure water (final concentration: 0.2 mM) was added dropwise on a beef liver (1 mm (length) x 1 mm (width) x 1 mm (height)). As a blank sample, ultra-pure water was used.

The anti-ice nucleation active performance (supercooling acceleration performance) was measured by using a method similar to the droplet-freezing method of Vali described in Test Example 1. The results are average values of the results of the test performed three times.

The results revealed that the anti-ice nucleation activity level of the anti-ice nucleation activator (tyrosine trimer) of Example 2 was 0.6°C, indicating that the tyrosine trimer of Example 2 exerted anti-ice nucleation activity.

Test Example 6: Anti-Ice Nucleation Activity Test With Respect to Biological Material (Tofu)

6-1: State of Tofu after Freezing and Thawing, as Well as Breaking Load of Tofu

Silken tofu (1.5 mm (length) x 1.5 mm (width) x 1.5 mm (height)) was immersed in the solution of Comparative Example 9 (water, blank) or Example 1 (tyrosine dimer) overnight. Thereafter, the tofu was taken out of the solution, the moisture was wiped off, and then the tofu was wrapped with wrap (plastic wrap). The tofu wrapped in plastic wrap was immersed in liquid nitrogen to be quickly frozen. The frozen tofu was then slowly thawed (warmed at 25°C) or quickly thawed (warmed at 60°C). Fig. 6 shows the state of cavities in the tofu after thawing (photographs) with the breaking load (N) of the tofu. The breaking load (N) of tofu was measured by a creep meter (RE2-33005C, Yamaden, Co., Ltd.).

Results

As shown in the results shown in Fig. 6, the breaking load of the tofu of Example 1 was low in both cases of slow thawing and quick thawing, compared to the breaking load of the tofu of Comparative Example 9. This indicates that no cavities were formed in the tofu of Example 1. It is thus confirmed that the tyrosine dimer of Example 1 exhibited anti-ice nucleation activity.

6-2. Observation of Tofu After One-Month Cryopreservation

The tofu quickly frozen as above (Examples 1 and 2, and Comparative Example 9) was cryopreserved at -20°C for 1 month. The frozen tofu was then slowly thawed (warmed at 25°C) or quickly thawed (warmed at 60°C). Fig. 7 shows the state (SEM photographs) of the tofu after thawing. Hereinbelow, the SEM photographs were taken by SEM (scanning microscope) (S-3000N, Hitachi, Ltd.). The white bar in the SEM photograph represents 100 μm.

Results

As shown in the photographs of Fig. 7, the tofu that was immersed in the solution of Example 1 (tyrosine dimer) or Example 2 (tyrosine trimer) maintained its dense-texture state, without the formation of cavities, in both cases of slow thawing and quick thawing, compared to the tofu that was immersed in the solution of Comparative Example 9 (blank).

6-3: Observation of Frozen Tofu

Silken tofu (1.5 mm (length) x 1.5 mm (width) x 1.5 mm (height)) was immersed overnight in the solution of Comparative Example 9 (blank) or Example 2 (tyrosine trimer), and slowly frozen at -20°C or quickly frozen in liquid nitrogen. Fig. 8 is SEM photographs showing the states of the frozen tofu. The white bar in each SEM photograph represents 100 μm.

Results

The tofu that was immersed in the solution of Example 2 (tyrosine trimer) maintained its dense-texture state when being frozen, without the formation of cavities, in both cases of slow freezing and quick freezing, compared to the tofu that was immersed in the solution of Comparative Example 9 (blank).

Test Example 7: Cooling Curve of Tofu

Silken tofu (1.5 mm (length) x 1.5 mm (width) x 1.5 mm (height)) that was immersed overnight in the solution of Comparative Example 9 (blank), Example 1 (tyrosine dimer), or Example 2 (tyrosine trimer) was cooled.

A thermocouple thermometer (Horiba) was inserted in tofu, and the temperature was measured every 20 seconds in a freezer at -20°C. A cooling curve was then obtained based on the relationship between the temperature (°C) and the cooling time (in seconds) (Fig. 9). Table 5 below and Fig. 9 show the temperature at which the tofu began to freeze (freezing onset temperature), the temperature at which the tofu was completely frozen, and the time taken for the tofu to be completely frozen.

Table 5

| | Freezing onset temperature | Temperature at which tofu was completely frozen | Time taken for tofu to be completely frozen |
|---|---|---|---|
| Blank (Comparative Example 9) | 0°C | -5°C | 780 seconds |
| Dimer (Example 1) | -1.2°C | -6.8°C | 1120 seconds |
| Trimer (Example 2) | -0.8°C | -7.8°C | 1080 seconds |

Results

Regarding the tofu containing the tyrosine dimer or tyrosine trimer of the present invention, the temperature at which the tofu began to freeze and the temperature at which the tofu was completely frozen were lower, and the time taken for the tofu to be completely frozen was longer, than the tofu that did not contain an anti-ice nucleation activator (Comparative Example 9). These results indicate that the tyrosine dimer and tyrosine trimer of the present invention exert anti-ice nucleation activity towards tofu.

Production Example 5 (P4VP (poly 4-vinylpyridine)-GGGYYY)

Fmoc-Tyr(tBu)-Alko-PEG Resin (0.200 mmol, 0.870 g) was placed in a column, and DMF as a reaction solvent

was added to the column, followed by stirring for 1 minute. This procedure was repeated 3 times. Next, methanol was added to the column, and the mixture was stirred for 1 minute, followed by washing. This procedure was repeated 3 times. Next, 25% DMSO/DMF was added to the column, and the mixture was stirred for 30 minutes to thus allow the mixture to be swollen. Thirty minutes later, DMF was added to perform stirring for 1 minute, and this procedure was repeated 3 times. Thereafter, 20% piperidine/DMF was added to the column, the mixture was stirred for 30 minutes, and Fmoc group deprotection was performed. Thirty minutes later, washing was performed with DMF and methanol. A small amount of the sample was added to a mixed solution of 20% TNBS, 40% PBS, and 40% $NaHCO_3$, and a color change was confirmed; this was considered to represent the completion of deprotection.

Next, swelling was performed for 30 minutes with 25% DMSO/DMF, followed by washing with DMF. Then, 0.600 mmol (0.276 g) of Fmoc-Tyr(tBu)-OH, 3.00 g of DMT-MM, 0.060 mL of NMM, and DMF were added to the column, followed by condensation for 2 hours. After condensation, washing was performed with DMF and methanol. Further, as with the confirmation of the Fmoc group deprotection described above, a small amount of the sample was added to the mixed solution, and no color change was observed; this was considered to represent the completion of condensation.

This procedure was repeated, and 0.600 mmol (0.178 g) of Fmoc-Gly-OH (Watanabe Chemical Industries, Ltd.) was used to synthesize the target peptide H-GGGYYY-Alko-PEG-Resin.

The synthesized H-GGGYYY-Alko-PEG-Resin and ACVA (0.600 mmol, 0.168 g), which is a polymerization initiator (Wako Pure Chemical Industries, Ltd.), were condensed for 3 hours in a similar manner. After condensation, washing was performed with DMF and methanol, and the resulting product was then swollen with DCM, followed by drying under reduced pressure. The ACVA-GGGYYY-Alko-PEG-Resin with which the initiator was condensed was added to DMF, a necessary amount of monomer 4-vinylpyridine (4-VP) (Wako Pure Chemical Industries, Ltd.), based on 1 equivalent amount of the peptide, was added thereto, and polymerization was performed in a water bath at 70°C under a nitrogen atmosphere while changing the polymerization time. After polymerization, the resulting product was placed into a column and washed with DMF and methanol, followed by drying under reduced pressure.

Next, a mixed solution of 0.5 mL of purified water and 9.5 mL of TFA was stirred in an ice bath for 10 minutes. The resulting mixed solution was taken out of the ice bath and added to the synthesized hybrid polymer, followed by stirring in a sample tube for 100 minutes. After stirring, diethyl ether was added thereto, and the resulting mixture was stirred in an ice bath for 10 minutes and crystallized. The precipitate was filtered off by suction and washed with diethyl ether. The precipitate was dissolved in 10% acetic acid and dialyzed. When a white precipitate was observed in the dialysis membrane, the solution was placed in a pear-shaped flask and lyophilized to obtain a target product.

Production Example 6 (P4VP-YYY)

P4VP-YYY was synthesized in a manner similar to the method described in Production Example 5, except that H-YYY-Alko-PEG-Resin was used in place of H-GGGYYY-Alko-PEG-Resin.

Production Example 7 (P4VP)

ACVA was dissolved in DMF (a reaction solvent), a necessary amount of monomer 4-VP, based on 1 equivalent amount of ACVA, was added thereto, and polymerization was performed at 70°C under a nitrogen atmosphere. The precipitate deposited with the addition of water was washed under suction filtration, dissolved in 10% acetic acid, and dialyzed. When a white precipitate was observed in the dialysis membrane, the solution was placed in a pear-shaped flask and lyophilized to obtain a target product.

Table 6 below shows the molecular weight of the compounds obtained in Production Examples 5 to 7. In the Test Examples below, the compounds shown in Table 6 were used, unless otherwise specified.

Table 6

| | Mn | Mw | Molecular weight distribution |
|---|---|---|---|
| P4VP | 21,000 | 30,000 | 1.5 |
| P4VP-GGGYYY | 23,000 | 36,000 | 1.6 |
| P4VP-YYY | 21,000 | 32,000 | 1.5 |
| Mn: Number average molecular weight<br>Mw: Weight average molecular weight | | | |

Test Example 8: Adsorptive Immobilization of Complex on Glass

Cover glasses with a diameter of 13 mm were immersed for 2 hours in a piranha solution comprising a mixture of concentrated sulfuric acid:30% hydrogen peroxide = 7:3 so as to remove the organic substances from the cover glass surface. Each cover glass was then washed with ultra-pure water and methanol, and placed in each well of a well plate, followed by drying under reduced pressure overnight.

The sample P4VP-GGGYYY (Mw: 36000, 46000, or 54000) to be adsorption-immobilized on the cover glass surface was dissolved in 10% acetic acid to a concentration of 1 mmol/L. Five hundred microliters of the resulting solution was dropped on each cover glass that was placed in each well of the well plate and dried under reduced pressure, for immersion overnight. One day later, the solution was sucked up, and the inside of each well of the well plate was washed with ultra-pure water 3 times. Subsequently, drying was performed again under reduced pressure.

To confirm adsorptive immobilization, the cover glass surface was analyzed using X-ray photoelectron spectroscopy (ESCA-3400HSE, Shimazu Corporation). As blank samples, two different types of cover glasses were used: a cover glass subjected to only piranha treatment; and a cover glass immersed in a solution obtained by dissolving, in 10% acetic acid, GGGYYY in place of P4VP-GGGYYY. Fig. 10 shows the results.

Results

According to the results of carbon ($C_{1s}$) shown in Fig. 10, P4VP-GGGYYY showed a larger peak, compared to the sample of piranha treatment only or the sample of the peptide only. According to the results of nitrogen ($N_{1s}$) shown in Fig. 10, no peaks were detected in the sample of piranha treatment only or the sample of the peptide only, while P4VP-GGGYYY showed a peak, which indicates that the complex was immobilized on the glass surface.

Example 11 and Comparative Example 10

An aqueous solution was prepared by dissolving P4VP-GGGYYY (Example 11) or glycine trimer (GGG) (Comparative Example 10) in ultra-pure water to a concentration of 1.0 mg/mL.

Test Example 9: Anti-Ice Nucleation Activity Test With Respect to Complex in Aqueous Solution

The anti-ice nucleation active performance of the complex in the aqueous solution was analyzed.

Nine hundred microliters of an aqueous solution was prepared by dissolving silver iodide in ultra-pure water to a silver iodide concentration of 1.0 mg/mL.

Each sample for evaluation was prepared by mixing 100 $\mu$L of the aqueous solutions of Examples 2 and 11 and Comparative Example 10 with 900 $\mu$L of the aqueous solution in which silver iodide was dissolved. A blank sample was prepared by mixing 100 $\mu$L of ultra-pure water with 900 $\mu$L of the aqueous solution in which silver iodide was dissolved.

The anti-ice nucleation active performance (supercooling acceleration performance) was measured by using the droplet-freezing method of Vali as described in Test Example 1. Fig. 11 shows the evaluation results regarding the anti-ice nucleation activators of Examples 2 and 11 and Comparative Example 10. The results are average values of the results of the test performed three times.

Results

The results indicate that P4VP-GGGYYY exerted anti-ice nucleation active performance in an aqueous solution.

Example 12 and Comparative Example 11

P4VP-YYY (Example 12) or P4VP (Comparative Example 11), in place of P4VP-GGGYYY, was immobilized on the cover glass surface in a manner similar to the method described in Test Example 8.

Test Example 10: Anti-Ice Nucleation Activity Test With Respect to Complex on Glass Surface

Four cover glasses each having a diameter of 13 mm and comprising P4VP (Comparative Example 11), P4VP-YYY (Example 12), or P4VP-GGGYYY (Example 13) immobilized on the glass surface were placed on an aluminum foil disposed on a cold plate chiller. Then, 100 $\mu$L of silver iodide aqueous solution (1.0 mg/mL) was taken with a Pipetman, and 3 drops per one cover glass (12 drops in total) were dropped. The temperature was gradually lowered under an electric current of 2A, and the temperature at which frost was observed in all of the 12 drops was considered to be the freezing point of the sample. The cover glass that was subjected to only piranha treatment was used as a blank. The

freezing point difference between the sample and the blank was considered to be the anti-ice nucleation activity level. Fig. 12 shows the results. The results are average values of the results of the test performed three times.

Results

P4VP-GGGYYY achieved the most excellent anti-ice nucleation active performance. This indicates that a longer peptide extending from the glass surface achieves a greater activity.

Industrial Applicability

The anti-ice nucleation activator of the present invention is widely applicable to food fields (e.g., quality preservatives for food, beverages, etc.); medical fields (e.g., cell preservatives, blood preservatives, and organ preservatives); cosmetic fields; environmental fields (e.g., coating compositions, agents for preventing frost damage, frost adhesion inhibitors); and the like.

## Claims

**1.** Use of a tyrosine peptide represented by Formula (1):

$$(X)_p\text{-}[Tyr]_n\text{-}(Y)_q \qquad (1)$$

wherein X and Y are identical or different, and each represents an amino acid residue,
n represents an integer of 2 to 5,
p represents an integer of 0 to 4, and
q represents an integer of 0 to 4,
with the proviso that p + q does not exceed 4,
wherein when p represents an integer of 2 to 4, two to four amino acid residues represented by $(X)_p$ may be identical or different, and
when q represents an integer of 2 to 4, two to four amino acid residues represented by $(Y)_q$ may be identical or different; or a complex composed of one or more of the tyrosine peptides and a polymer, wherein the one or more of the tyrosine peptides are bonded to the polymer
as an anti-ice nucleation activator.

**2.** The use according to claim 1, wherein n represents an integer of 2 to 4.

**3.** The use according to claim 1 or 2, wherein n represents 2 or 3, p represents 0 or 1, and q represents 0 or 1.

**4.** Use of a composition comprising the tyrosine peptide as defined in any one of claims 1 to 3, and water as an antifreeze liquid.

**5.** Use of a coating solution comprising the complex as defined in claim 1 as an anti-frost damage agent.

**6.** A method for improving the anti-ice nucleation activity of a biological material, the method comprising the step of bringing the anti-ice nucleation activator as defined in any one of claims 1 to 3 into contact with a biological material.

**7.** A method for preserving a biological material in vitro, the method comprising the step of bringing the anti-ice nucleation

activator as defined in any one of claims 1 to 3 into contact with a biological material.

## Patentansprüche

1. Verwendung eines Tyrosinpeptids, dargestellt durch Formel (I):

$$(X)_p\text{-}[Tyr]_n\text{-}(Y)_q \qquad (1)$$

worin X und Y identisch oder verschieden sind und jeweils einen Aminrest darstellen,
n eine ganze Zahl von 2 bis 5 darstellt,
p eine ganze Zahl von 0 bis 4 darstellt und
q eine ganze Zahl von 0 bis 4 darstellt,
mit der Maßgabe, dass p + q nicht größer ist als 4,
wobei, wenn p eine ganze Zahl von 2 bis 4 darstellt, zwei bis vier Aminosäurereste, die durch $(X)_p$ dargestellt werden, identisch oder verschieden sein können, und,
wenn q eine ganze Zahl von 2 bis 4 darstellt, zwei bis vier Aminosäurereste, die durch $(Y)_q$ dargestellt werden, identisch oder verschieden sein können; oder
eines Komplexes, der aus einem oder mehreren der Tyrosinpeptide und einem Polymer besteht, wobei der eine oder die mehreren der Tyrosinpeptide an dem Polymer gebunden ist/sind,
als einen Nukleierungsaktivator mit Gefrierschutz gegen Eisbildung.

2. Verwendung nach Anspruch 1, wobei n eine ganze Zahl von 2 bis 4 darstellt.

3. Verwendung nach Anspruch 1 oder 2, wobei n 2 oder 3 darstellt, p 0 oder 1 darstellt und q 0 oder 1 darstellt.

4. Verwendung einer Zusammensetzung, umfassend das Tyrosinpeptid, wie in einem der Ansprüche 1 bis 3 festgelegt, sowie Wasser als Gefrierschutzflüssigkeit.

5. Verwendung einer Beschichtungslösung, umfassend den Komplex, wie unter Anspruch 1 festgelegt, als ein Mittel gegen Frostschäden.

6. Verfahren zum Verbessern der Anti-Eiskristallkeimbildungsaktivität eines biologischen Materials, wobei das Verfahren den Schritt umfasst, den Anti-Eiskristallkeimbildungsaktivator nach einem der Ansprüche 1 bis 3 mit einem biologischen Material in Kontakt zu bringen.

7. Verfahren zum Konservieren eines biologischen Materials in vitro, wobei das Verfahren den Schritt umfasst, den Nukleierungsaktivator mit Gefrierschutz gegen Eisbildung, wie in einem des Ansprüche 1 bis 3 definiert, mit einem biologischen Material zu bringen.

## Revendications

1. Utilisation d'un peptide de tyrosine représenté par la Formule (1) :

$$(X)_p\text{-}[Tyr]_n\text{-}(Y)_q \qquad (1)$$

dans laquelle X et Y sont identiques ou différents, et chacun représente un résidu d'acide aminé,
n représente un entier de 2 à 5,
p représente un entier de 0 à 4, et
q représente un entier de 0 à 4,
avec pour condition que p + q ne dépasse pas 4,
dans laquelle lorsque p représente un entier de 2 à 4, deux à quatre résidus d'acides aminés représentés par $(X)_p$ peuvent être identiques ou différents, et
lorsque q représente un entier de 2 à 4, deux à quatre résidus d'acides aminés représentés par $(Y)_q$ peuvent être identiques ou différents ; ou
ou d'un complexe composé d'un ou plusieurs des peptides de tyrosine et d'un polymère, dans lequel le ou les peptides de tyrosine sont liés au polymère en tant qu'activateur anti-glaciogène.

2. Utilisation selon la revendication 1, dans laquelle n représente un entier de 2 à 4.

3. Utilisation selon la revendication 1 ou 2, dans laquelle n représente 2 ou 3, p représente 0 ou 1, et q représente 0 ou 1.

4. Utilisation d'une composition comprenant le peptide de tyrosine selon l'une quelconque des revendications 1 à 3, et de l'eau en tant que liquide antigel.

5. Utilisation d'une solution de revêtement comprenant le complexe selon la revendication 1 en tant qu'agent anti-endommagement par le givre.

6. Procédé pour améliorer l'activité de nucléation antigel d'un matériau biologique, le procédé comprenant l'étape consistant à mettre l'activateur de nucléation antigel selon l'une quelconque des revendications 1 à 3 en contact avec une matière biologique.

7. Procédé pour conserver une matière biologique *in vitro,* le procédé comprenant l'étape consistant à mettre l'activateur de nucléation antigel selon l'une quelconque des revendications 1 à 3 en contact avec une matière biologique.

Fig. 1

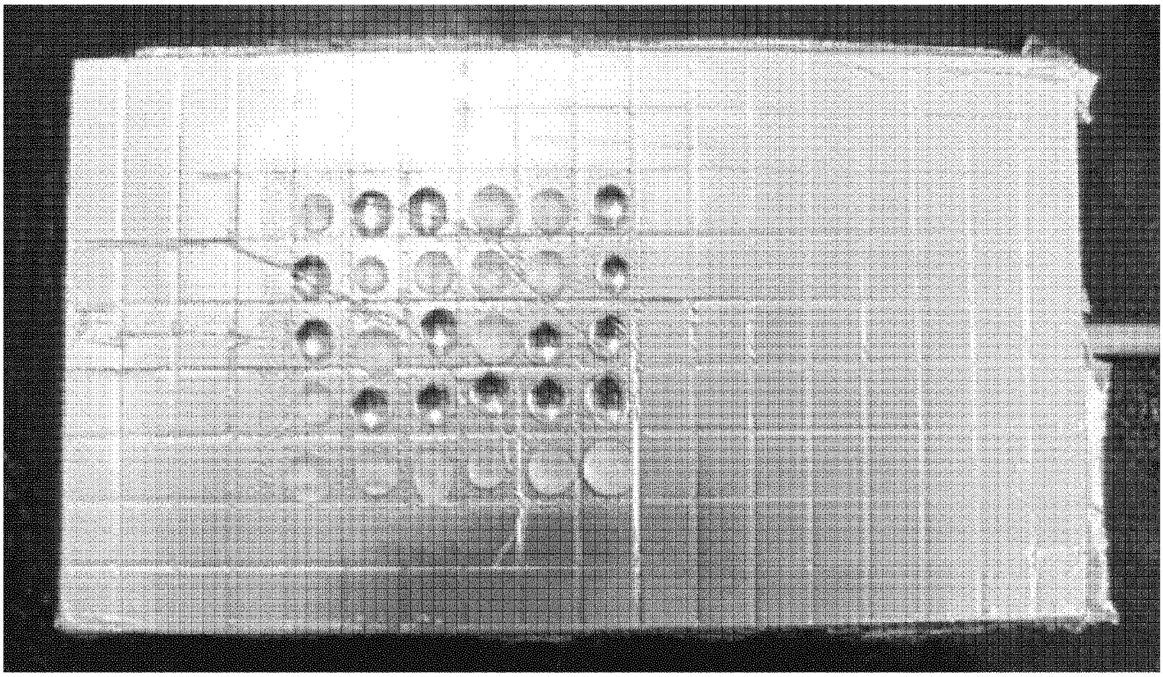

Fig. 2

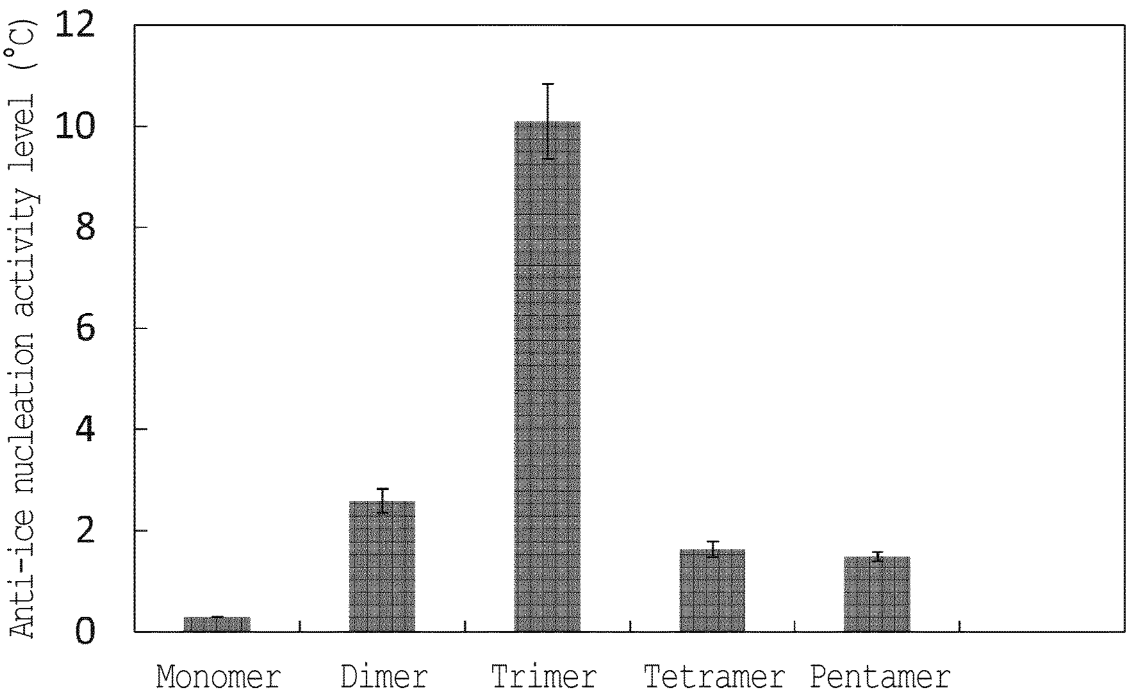
Anti-ice nucleation activity level (°C)
0
2
4
6
8
10
12
Monomer
Dimer
Trimer
Tetramer
Pentamer

Fig. 3

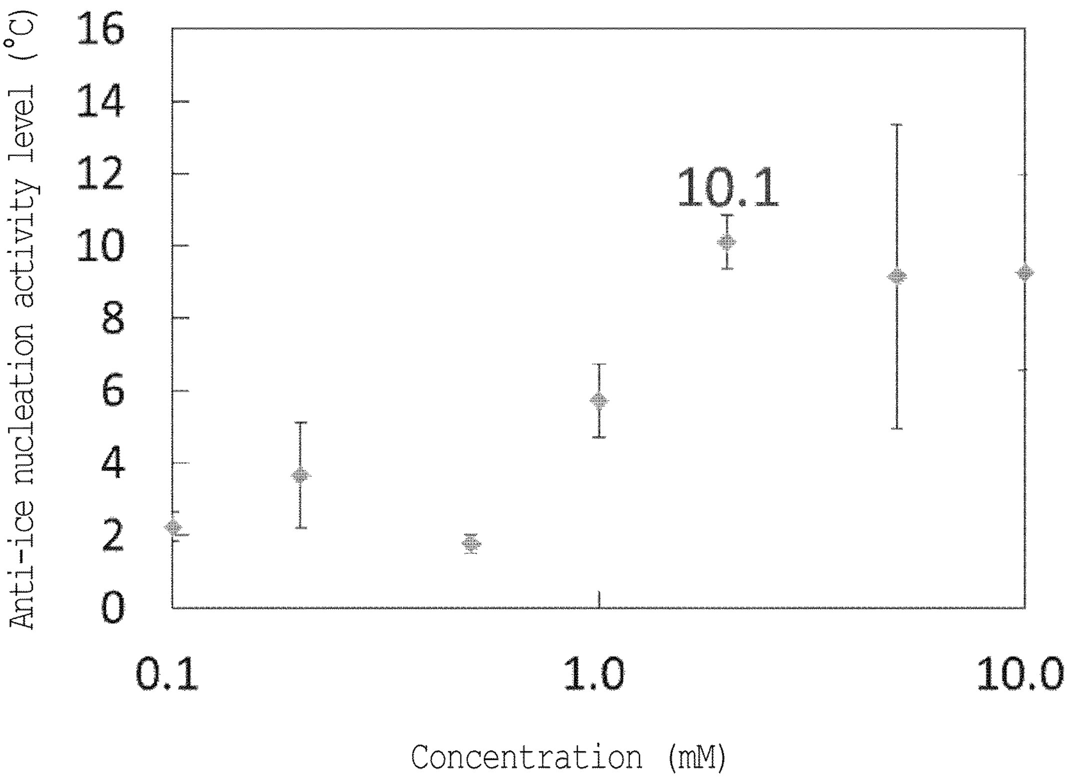
Anti-ice nucleation activity level (°C)
16
14
12
10
8
6
4
2
0
10.1
0.1
1.0
10.0
Concentration (mM)

Fig. 4

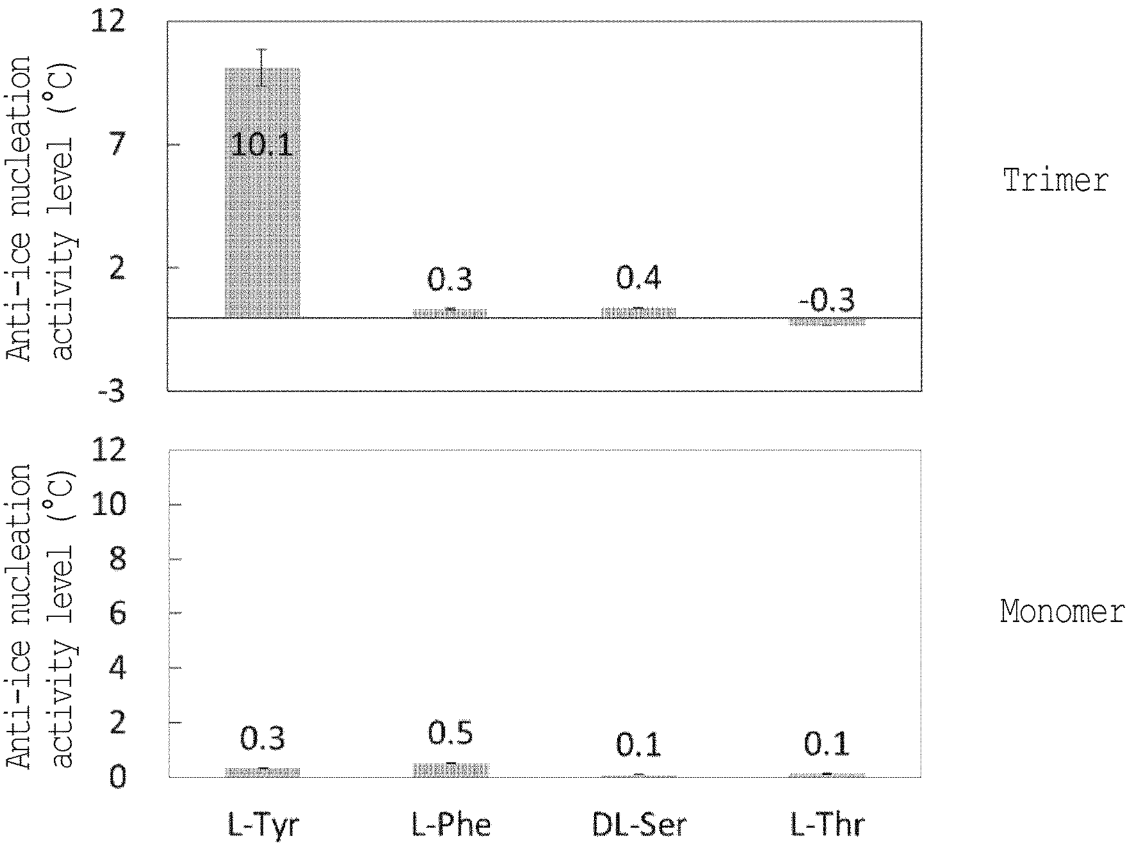
12
7
2
-3
Anti-ice nucleation activity level (°C)
10.1
0.3
0.4
-0.3
Trimer
12
10
8
6
4
2
0
Anti-ice nucleation activity level (°C)
0.3
0.5
0.1
0.1
Monomer
L-Tyr
L-Phe
DL-Ser
L-Thr

Fig. 5

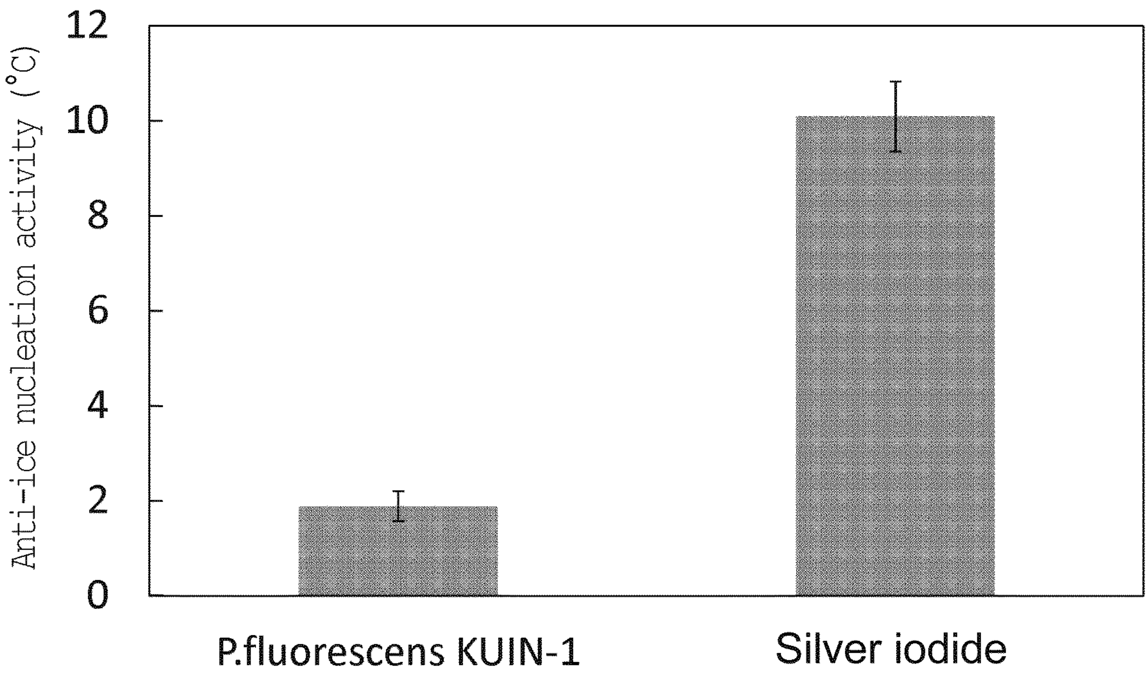


Fig. 6

| Anti-ice nucleation activator-containing solution | Quick freezing | |
|---|---|---|
| | Slow thawing | Quick thawing |
| Comparative Example 9<br>Blank | 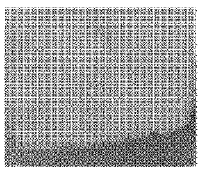 | 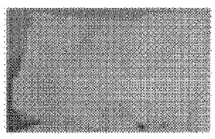 |
| Breaking load | 2.6 N | 19.1 N |
| Example 1<br>Tyrosine Trimer (2 mM) | 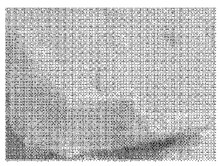 | 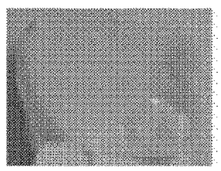 |
| Breaking load | 0.9 N | 2.6 N |

Fig. 7

| Solution for immersion | Quick freezing | |
|---|---|---|
| | Slow thawing | Quick thawing |
| Blank | 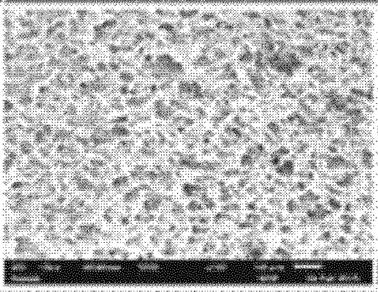 | 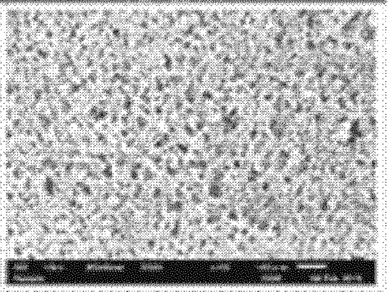 |
| Dimer 2 mM | 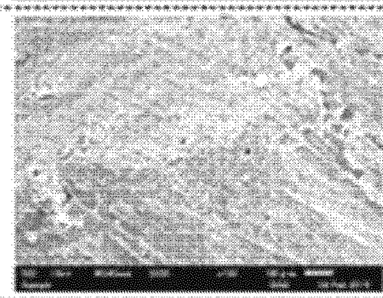 | 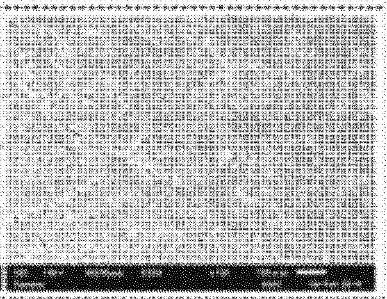 |
| Trimer 2 mM | 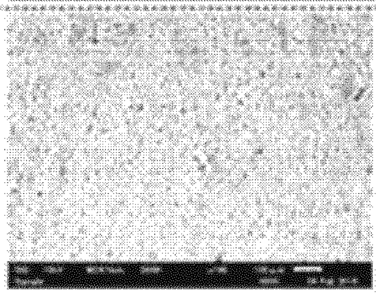 | 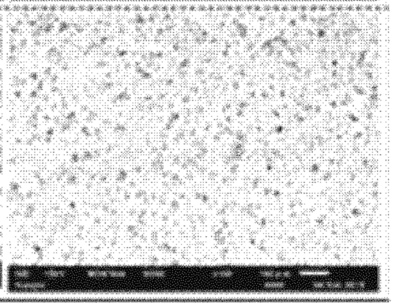 |

Fig. 8

| Solution for immersion | Slow thawing (-20°C) | Quick thawing (Liquid nitrogen) |
|---|---|---|
| Blank | 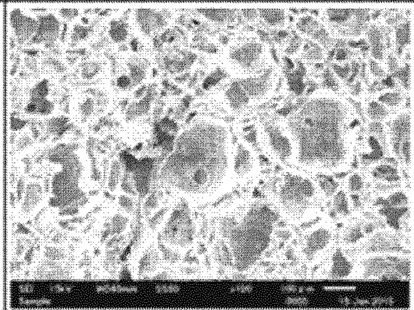 | 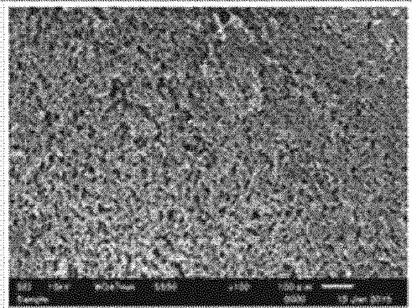 |
| Tyrosine trimer 0.1% | 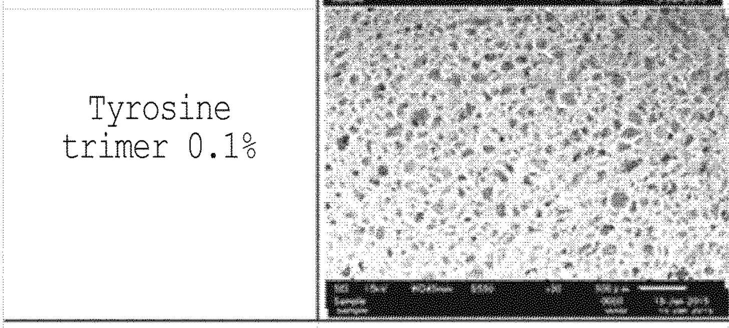 | 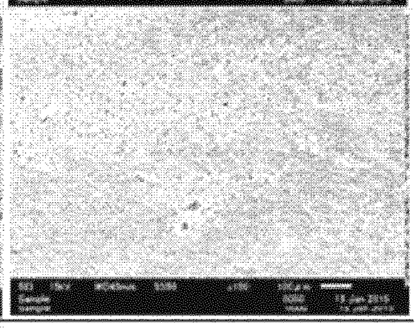 |

Fig. 9

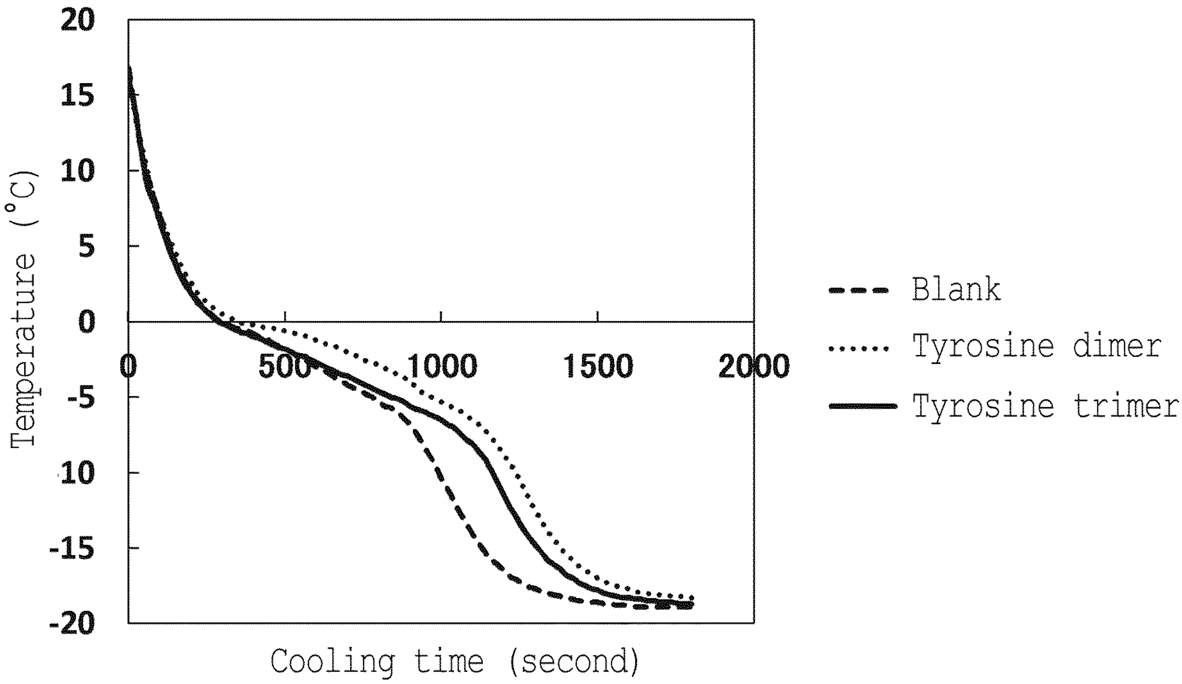
20
15
10
5
0
-5
-10
-15
-20
Temperature (°C)
0
500
1000
1500
2000
Cooling time (second)
Blank
Tyrosine dimer
Tyrosine trimer

Fig. 10

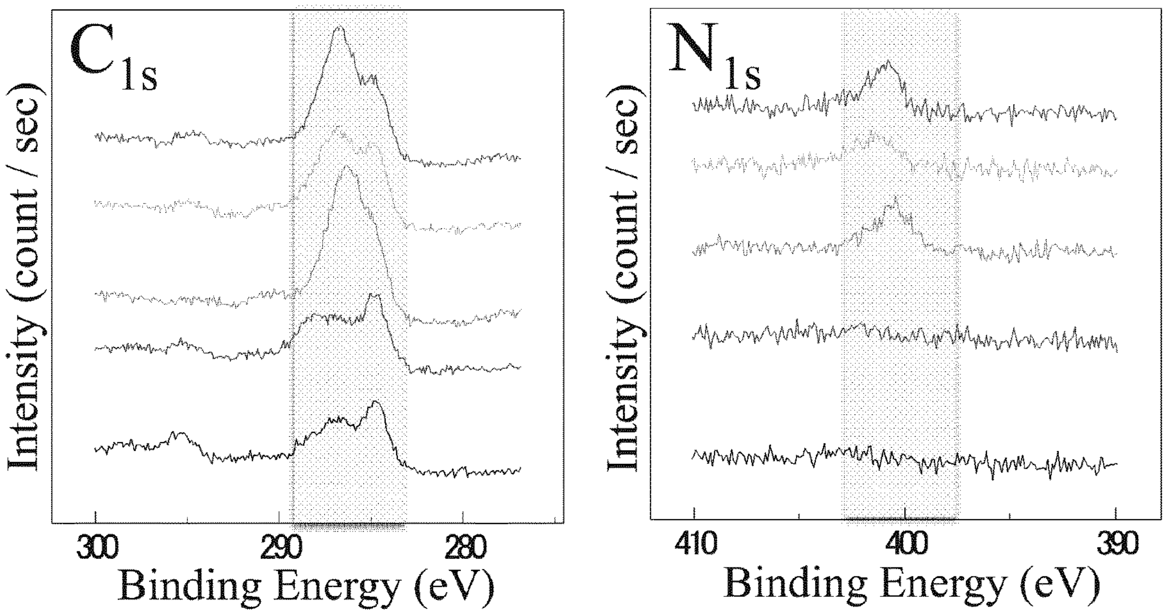
C1s
Intensity (count / sec)
300
290
280
Binding Energy (eV)
N1s
Intensity (count / sec)
410
400
390
Binding Energy (eV)

Fig. 11

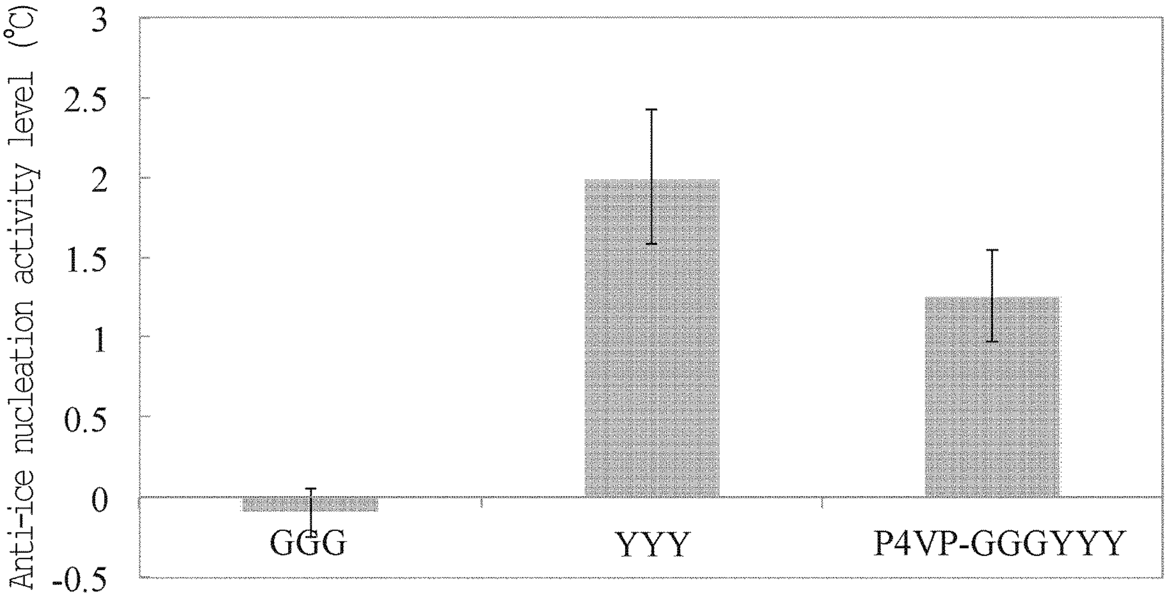
Anti-ice nucleation activity level (℃)
3
2.5
2
1.5
1
0.5
0
-0.5
GGG
YYY
P4VP-GGGYYY

Fig. 12

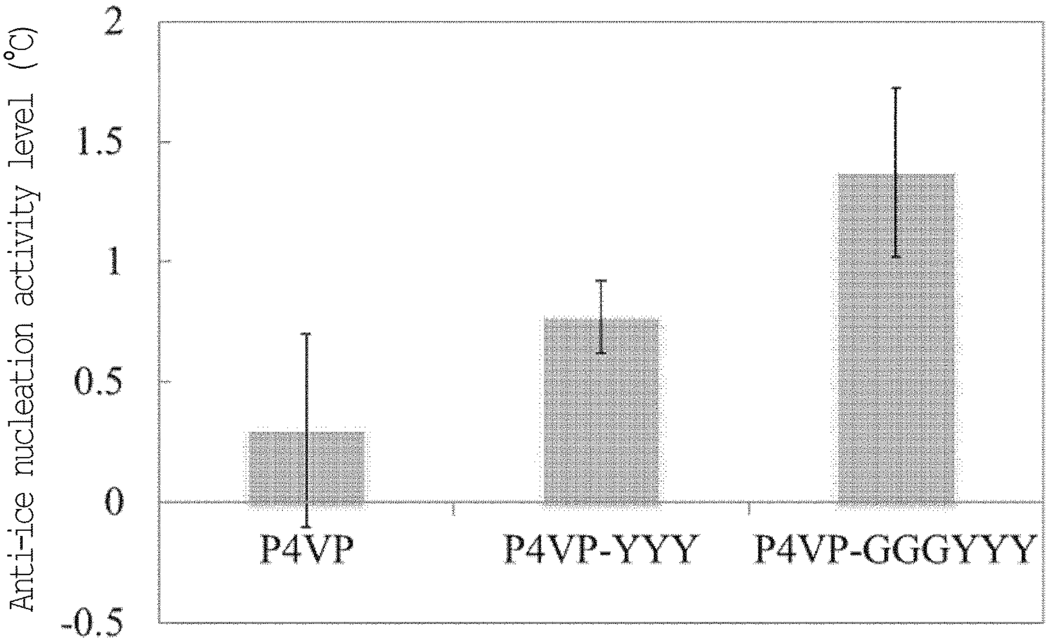
Anti-ice nucleation activity level (°C)
2
1.5
1
0.5
0
-0.5
P4VP
P4VP-YYY
P4VP-GGGYYY

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010121052 A
- JP 2015095251 A


### Non-patent literature cited in the description


- **H. KAWAHARA et al.** *J. Antibact. Antifung. Agents,* 1996, vol. 24, 95-100
- **Y. YAMASHITA et al.** *Biosci. Biotech. Biochem.,* 2002, vol. 66, 948-954
- IUPAC-IUB Communication on Biological Nomenclature. *Eur. J. Biochem.,* 1984, vol. 138, 9

SEQUENCE LISTING

<110> THE SCHOOL CORPORATION KANSAI UNIVERSITY

<120> ANTI-ICE NUCLEAR ACTIVITY AGENT

<130> P16-073WO

<150> JP 2015-095251
<151> 2015-05-07

<160> 4

<170> PatentIn version 3.5

<210> 1
<211> 4
<212> PRT
<213> Artificial Sequence

<220>
<223> tyrosine peptide

<400> 1

```
Tyr Tyr Tyr Tyr
1
```

<210> 2
<211> 5
<212> PRT
<213> Artificial Sequence

<220>
<223> tyrosine peptide

<400> 2

```
Tyr Tyr Tyr Tyr Tyr
1               5
```

<210> 3
<211> 6
<212> PRT
<213> Artificial Sequence

<220>
<223> tyrosine peptide

<400> 3

```
Tyr Tyr Tyr Tyr Tyr Tyr
1               5
```

<210> 4
<211> 6
<212> PRT
<213> Artificial Sequence

<220>
<223> tyrosine peptide

<400> 4

```
Gly Gly Gly Tyr Tyr Tyr
1               5
```